(19)

**Europäisches Patentamt**

**European Patent Office**

**Office européen des brevets**

(11) **EP 4 340 721 B1**

(12) **EUROPEAN PATENT SPECIFICATION**

(45) Date of publication and mention
of the grant of the patent:
**22.04.2026 Bulletin 2026/17**

(21) Application number: **22730433.4**

(22) Date of filing: **23.05.2022**

(51) International Patent Classification (IPC):
**A61B 5/1455** (2006.01)    **A61B 5/00** (2006.01)
**G01L 11/02** (2006.01)    **F21V 8/00** (2006.01)

(52) Cooperative Patent Classification (CPC):
**A61B 5/447; A61B 5/14552; A61B 5/6804;**
**G01L 11/025;** A61B 2562/0233; A61B 2562/0247;
A61B 2562/046; A61B 2562/06; G02B 6/001

(86) International application number:
**PCT/EP2022/063894**

(87) International publication number:
**WO 2022/243564 (24.11.2022 Gazette 2022/47)**

(54) **MEDICAL DEVICE COMPRISING A WEARABLE TEXTILE SENSOR TO PROTECT AGAINST PRESSURE INJURIES**

MEDIZINISCHE VORRICHTUNG MIT EINEM AM KÖRPER TRAGBAREN TEXTILSENSOR ZUM SCHUTZ VOR DRUCKVERLETZUNGEN

DISPOSITIF MÉDICAL COMPRENANT UN CAPTEUR DE TEXTILE PORTABLE POUR PROTÉGER CONTRE LES BLESSURES DUES À LA PRESSION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **21.05.2021 EP 21175481**

(43) Date of publication of application:
**27.03.2024 Bulletin 2024/13**

(73) Proprietors:
• **Universität Bern**
  **3012 Bern (CH)**
• **EMPA Eidgenössische Materialprüfungs- und
  Forschungsanstalt
  8600 Dübendorf (CH)**

(72) Inventors:
• **WOLF, Ursula**
  **8038 Zürich (CH)**
• **DA SILVA-KRESS, Oliver**
  **3172 Niederwangen (CH)**
• **CANTIENI, Tarcisi**
  **3012 Bern (CH)**
• **BOESEL, Luciano**
  **9008 St. Gallen (CH)**

• **ROSSI, René**
  **9500 Wil SG (CH)**
• **ANSARI, Nazanin**
  **78462 Konstanz (DE)**
• **MICHLER, Markus**
  **6800 Feldkirch (AT)**

(74) Representative: **Schulz Junghans
Patentanwälte PartGmbB
Großbeerenstraße 71
10963 Berlin (DE)**

(56) References cited:
• **QUANDT BRIT M ET AL: "Flexible POFF sensors
  for decubitus prevention", 1 January 2015
  (2015-01-01), pages 1 - 1, XP055857598,
  Retrieved from the Internet <URL:http://www.
  nanotera.ch/pdf/posters2015/ParaTex187.pdf>
  [retrieved on 20211103]**
• **QUANDT BRIT MAIKE: "Optical Fibre Textiles in
  Non-Invasive Medical Applications", 1 January
  2016 (2016-01-01), XP055857629, Retrieved from
  the Internet <URL:https://www.
  research-collection.ethz.ch/handle/20.500.11850/
  156225> [retrieved on 20211103], DOI: 10.3929/
  ethz-a-010852606**

EP 4 340 721 B1

**Description**

The present invention relates to a medical device for determining an oxygen saturation in a tissue of a human or animal subject. Furthermore, the invention relates to a corresponding method.

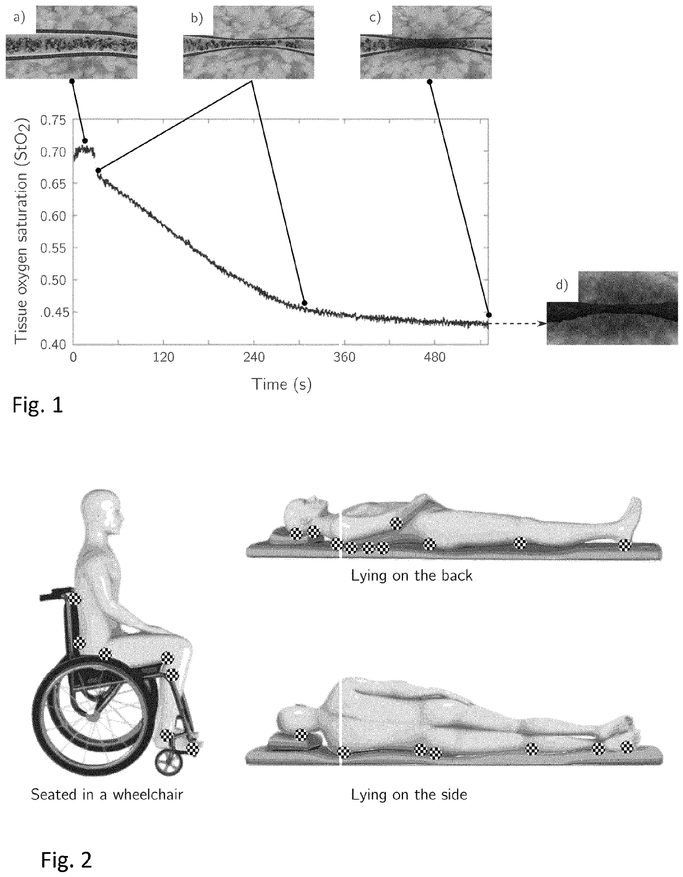

Pressure injuries (PI) occur in humans and animals when a region of tissue becomes poorly oxygenated (due to localized pressure acting on the tissue) for a duration long enough to induce tissue necrosis. Tissue oxygen saturation ($StO_2$) can drop rapidly with pressure and the onset of necrosis can set in within hours. The rate of decrease of $StO_2$ with time due to external pressure is shown in Fig. 1. These injuries often begin to form below the skin and grow substantially before becoming visible. Though the lack of oxygen is very painful to many, a great number of people are afflicted due to the inability to sense the presence of such an injury, as is the case with individuals with a damaged spinal cord or otherwise compromised nervous system such as paraplegics. In such cases, the subdermal wounds are allowed to grow, unnoticed, until they erupt at the surface and pose great health risks. PI are divided into four stages. The first stage is redness and tenderness at the site where the injury develops. The second stage is the formation of an open wound. The third and fourth stages are advanced tissue degeneration.

At these advanced stages, the healing process becomes complicated and may take many months, causing great discomfort to the affected individual. The leading groups of individuals susceptible to PI are paraplegics (20-40%), ICU patients (8-40%), patients who have undergone surgery (4-21%) and the elderly (less than 30%). PI form in areas of high pressure, such as the sacrum (lower back) or under the hips (ischial tuberosity) in the seated position or the regions of the head, shoulders, hips, knees and feet in the lying position where the cause of the localized high pressure is often a bony prominence or pointed bones deep below the surface of the skin. (cf. Fig. 2).

The prior art attempts to prevent pressure injuries by using cushioned seats and beds which evenly distribute or alter the pressure applied to the individual in a way to stimulate blood flow in tissue regions susceptible to the formation of pressure injuries. Other prior art includes motion monitoring or pressure monitoring as a reminder for caregivers to move patients. The prior art also includes measures taken upon an individual who is required to be seated or lying down for prolonged periods.

These measures include the individual being moved by an assistant, or being instructed to move themselves, at time intervals known to be critically long periods. This includes asking paraplegic to lift themselves from their wheelchairs every 10 minutes for a duration of one minute to allow blood to reintroduce oxygen to the tissue under the ischial tuberosity in order to prevent pressure injuries.

The most common method of preventing PI today is for the patient or caregiver to follow a strict protocol of regularly moving the patient at intervals that can be as short as every ten minutes and are usually in the range from 2 to 4 hours while conducting regular skin checks to look for signs of the first stage of PI formation (redness or swelling). These checks are additionally performed whenever the patient acquires a new bed, wheelchair, after a long journey or after sports. These checkups are inconvenient and personally invasive for the patient and the caregiver. Further methods of prevention have been developed in the form of mattresses which change the pressure distribution over time to help stimulate blood flow and seat cushions in wheelchairs that measure the seated pressure. Because the direct relation between true tissue oxygen saturation and pressure at the surface of the skin remains unknown, the method of only measuring pressure cannot adequately be used for prevention of PI.

Particularly, it has been proposed to use optical fibers to allow light to be coupled into human tissue on which the fibers are placed and collected again from the human tissue at another location at the surface in order to measure changes in tissue oxygenation by means of near-infrared spectroscopy. The prior art therefore provides a means of a near-infrared sensor which can be worn against the skin. The shortcomings of the prior art for the purpose of a monitoring system which allow for targeted interventional movement to assist in the prevention of pressure injuries, is the lack spatial and depth resolution to allow detection of tissue oxygenation changes precisely at locations of bony prominences or pointed bone segments deep beneath the skin surface where the tissue necrosis responsible for the onset of pressure injuries begins.

Reference documents [3] and [4] disclose flexible textiles comprising a network of optical fibers to determine oxygen saturation.

Therefore, based on the above, the problem to be solved by the present invention is to provide an improved medical device and method of detecting the physiological changes in the tissue which indicate the possible onset of a pressure injury.

The problem is solved by a medical device having the features of claim 1 and a method having the features of claim 15.

Preferred embodiments of these aspects of the present invention are stated in the corresponding sub claims and are also described below.

According to claim 1, a medical device for determining an oxygen saturation in a tissue of e.g. a human or an animal subject is disclosed, comprising:

-    a sensor comprising a flexible textile preferably configured to be arranged and in contact with the subject's skin, the textile comprising a plurality of preferably polymer-based first optical fibers and a plurality of preferably polymer-based second optical fibers, wherein the first optical and second optical fibers each comprise a first end and a second end,

- a first light generating unit configured to generate and couple light of a first wavelength into the first ends of the first optical fibers and to generate and couple light of a second wavelength into the second ends of the first optical fibers, the first wavelength being different from the second wavelength, wherein the respective first optical fiber is configured to emit light coming from the first light generating unit via a light emitting section of the respective first optical fiber to irradiate said tissue of the subject, the light emitting section being arranged between the first end and the second end of the respective first optical fiber, and wherein the respective second optical fiber comprises a light receiving section configured to receive light coming from the tissue, the light receiving section being arranged between the first end and the second end of the respective second optical fiber,

- wherein the sensor further comprises a first light detector unit operatively connected to the second optical fibers and configured to detect light received by the light receiving section of the respective second optical fiber, and

- an optional analyzing unit configured to determine an oxygen saturation value of the tissue using intensities of light detected by the first light detector unit.

Particularly, in the framework of the present invention, flexible textile means, that the textile is pliable so as to allow the textile to conform to a curvature of the surface of a subject's skin and, in particular, to fit snugly against it. The flexible textile can be formed by any suitable method such as weaving, knitting, crocheting, knotting, tatting, felting, bonding, or braiding. Particularly the first and second optical fibers can be integrated into the flexible textile by any suitable method such as stitching or embroidering. As detailed further below, the flexible textile, that is also denoted as host textile herein, can comprise supporting fibers which form a base of the flexible textile into which the optical fibers are integrated. The supporting fibers can further form yarns or threads of the textile or any other suitable elongated element from which the textile can be formed (e.g. by the above-stated methods).

According to a preferred embodiment of the present invention, the medical device the light emitting sections of the first fibers are arranged to form a plurality of separate light emitting regions on the flexible textile, each light emitting region comprising a plurality of light emitting sections of a corresponding number of first fibers. According to yet another preferred embodiment, the light receiving sections of the second fibers are arranged to form a plurality of separate light receiving regions on the flexible textile, each light receiving region comprising a plurality of light receiving sections of a corresponding number of second fibers.

Particularly, the light emitting regions can form localized point-like light sources whereas the light receiving regions can form localized point-like light detectors.

According to a preferred embodiment of the present invention, a distance between a light emitting region and a light receiving region is at least 5mm, particularly at least 10 mm, particularly at least 20 mm. According to yet another preferred embodiment, a distance between any light emitting region and light receiving region (between which a region of interest to be measured exists) is in the range from 8 mm to 60 mm. According to yet another embodiment, a diameter of each light emitting region is in the range from 3 mm to 10 mm, and/or wherein a diameter of each light receiving region is in the range from 3 mm to 10 mm. Particularly, the respective diameter can be about 6mm. Here diameter particularly means the diameter of a minimal periphery that surrounds all light emitting or light receiving sections of the respective light emitting or receiving region.

In an embodiment, instead of measuring merely an oxygenation saturation value of the tissue, the analyzing unit is configured to determine an oxygen saturation distribution of the tissue using intensities of near infrared light detected by the first light detector unit, the oxygen saturation distribution assigning to each of a plurality of points in said tissue an oxygen saturation value of the tissue.

Particularly, said plurality of points exist due to the fact that the light emitting regions and the light receiving regions on the flexible textile define a plurality of distances between light emitting and light receiving regions between which light can travel and which allow for a measurement volume of an underlying tissue of a person to be measured throughout the measurement volume.

Advantageously, due to being able to illuminating each first fiber from both ends, each light emitting section (e.g. corresponding loop(s)) can emit two wavelengths. This allows one to emit multiple wavelengths from the same location which is beneficial for multi-wavelength calculations, which usually requires that the same portion of tissue is illuminated and that the distance from the source location to the detector location is the same for both wavelengths.

Furthermore, due to said light emitting (or receiving) regions, not only a single fiber which is e.g. fixated by three loops (the light emitting section) can be utilized, but each separated/localized light emitting region can be comprised of multiple first fibers, each with a light emitting section of e.g. three loops, to form a dense collection of light emitting sections. Preferably, the light emitting regions effectively form point emitters allowing a multi-distance, spatial resolution approach to work.

Furthermore, according to an embodiment, each light emitting region comprises a further plurality of first optical fibers, each first optical fiber of said further plurality comprising a first end and a second end, wherein the first light generating unit is configured to generate and couple light of a third wavelength into the first ends and to generate and couple light of a fourth wavelength into the second ends, the third wavelength being different from the fourth wavelength (the first, second, third and fourth wavelength being different from one another in particular). Furthermore, particular, the respective first optical fiber of said further plurality is configured to emit light via a light emitting section of the respective first optical fiber of said further plurality to irradiate tissue of the subject, the light emitting section being arranged between the first end and the second end of the respective first optical fiber of said further plurality of first optical fibers.

Correspondingly, according to a further embodiment of the invention, each light receiving region comprises a further plurality of second optical fibers, each second optical fiber comprising a first end and a second end, wherein the respective second optical fiber of said further plurality of second optical fibers comprises a light receiving section configured to receive light coming from the tissue, the light receiving section being arranged between the first end and the second end of the respective second optical fiber of said further plurality of second optical fibers. Furthermore, particularly, the first light detector unit is operatively connected to the second optical fibers of said further plurality of second optical fibers.

Thus, in other words, a second set of optical fibers allowing to apply two wavelengths can be arranged in close enough proximity, densely enough, so that each light emitting region actually comprises two (or more) sets of light emitting sections for illuminating with four different wavelengths from a single localized light emitting region. Likewise, the device also comprises a multitude of multi-fiber light receiving regions, i.e. detection points.

Thus, particularly, the present invention provides separate localized light emitting regions that can each emit at least two, particularly four (or even more) wavelengths into the tissue area of interest. Particularly, due to having a plurality of distributed light emitting and receiving regions a 3D oxygen saturation distribution can be determined for the measuring volume of interest in the tissue.

In this way, the present invention advantageously provides a medical device (and a corresponding method to be described further below), which particularly measures the physiological changes of a person's tissues, continuously, for prolonged periods, while being portable and while not, itself, introducing pressure points to the susceptible tissue which is to be monitored. The present invention therefore provides a practical solution to the current lack of methods or devices capable of such detection and prevention of pressure injuries. These physiological changes include, but are not limited to, changes in tissue oxygen saturation as calculated by the relative or absolute changes in the chromophores oxy-hemoglobin and deoxy-hemoglobin by measuring the absorption of near-infrared light (NIR). Optionally, also integrated into the sensor is a pressure sensor which maps the pressure at the skin's surface. Furthermore, in an embodiment, the medical device can also be configured to conduct photoplethysmography.

Near-infrared spectroscopy (NIRS) has been used to measure various physiological properties in animal and human subjects. An important parameter has been the measurement of hemoglobin in the blood and in various tissue types. The present invention solves the presented problem by using the concept of near-infrared spectroscopy to measure these physiological changes in regions of tissue susceptible to pressure injuries in a non-invasive way without itself introducing pressure on the tissue, particularly by using the spatially resolved, multi-distance, and multi-wavelength spectroscopy based on the sensor according to the present invention.

The prior art only allows for the measurement of tissue oxygen saturation or applied pressure at the tissue surface, but not both at once. Furthermore, pressure sensors are often bulky, often creating pressure points themselves. The direct relation between the pressure exerted on tissue and the resulting change in tissue oxygen saturation is unknown. In embodiments described further below, the present invention allows both measurements to take place concurrently, so that a direct relation between pressure and tissue oxygenation can be measured.

According to an embodiment of the medical device according to the present invention, the medical device further comprises a control unit operatively connected to the first light generating unit and the first light detector unit, the control unit configured to control [cause/activate/trigger] generation of said near infrared light by the first light generating unit and to generate first raw data indicative of intensities of near infrared light detected by the first light detector unit.

Further, in an embodiment of the medical device, the flexible textile is configured to be placed on a skin portion of the subject to contact the skin portion, the skin portion forming an outer surface of a volume occupied by the tissue.

Further, in an embodiment of the medical device, the medical device comprises a garment configured to be worn by a subject, the garment comprising an inside, the flexible tissue forming at least an integral portion of said inside. Alternatively, the flexible tissue can be configured to be releasably connected to the garment.

According to a preferred embodiment, the garment is one of: a sock (particularly a pair of socks, each sock comprising a sensor according to the present invention), underpants, leggings, long sleeved shirt, a sports clothing, an undershirt, a cap configured to be worn on the head of the subject.

Thus, the invention particularly allows to provide persons who are in danger of developing pressure injuries or other complications which may follow from changes in tissue oxygenation unable to otherwise be monitored due to current sensors not being wearable in the sense that they are not effectively indistinguishable from a textile or garment which has no local pressure points or causes of discomfort, a wearable flexible sensor, particularly in combination with a textile or garment, which monitors the tissue oxygen saturation in regions and spatially resolved depths onto which forces and pressure are exerted in real-time and to give the wearer of the garment or textile direct feedback of the tissue oxygenation in the region of interest by means of a visualization or of an alarm which alerts them of critically low oxygenation, prompting them to move themselves or to be moved by an assisting person. Particularly, this garment, by way of its design being based on a flexible textile, does not cause additional pressure points by itself. The present invention therefore particularly seeks to prevent injury and pain for subjects prone to pressure injuries while easing the financial burden of treating the millions of cases of PI every year worldwide.

Further, according to an embodiment of the medical device, the medical device comprises a housing enclosing the control unit, the first light generating unit, and the first light detector unit.

Preferably, in an embodiment, the housing of the control unit is configured to be fastened to the subject at a convenient location such as the front of the waistband so as to not cause disruption to the subject's movements, pain, or other discomfort while being worn for extended periods.

Furthermore, according to an embodiment, the control unit is configured to transmit said first raw data to the analyzing unit.

Further, according to an embodiment of the medical device, the control unit is operatively connected to the analyzing unit via a wireless communication connection or a cable to transmit said first raw data to the analyzing unit. Further, according to an embodiment of the medical device, the light emitting section of the respective first fiber is a curved light emitting section comprising a curvature adapted such that the light emitting section of the respective first optical fiber emits near infrared light. Particularly, the respective light emitting section can comprises one of: at least one loop (e.g., one, two, three, or even more loops), a knot (e.g., one, two, three, or even more knots), a bend. Particularly, the respective curved structure (loop, knot, bend, etc.) is formed such that said curvature of the optical fiber exceeds a critical curvature required for radiation to escape the optical fiber via bending loss. Particularly, also other topologies can be used that are appropriate for textile integration without inducing local pressure points on the patient's skin and allow to emit near infrared light from the first optical fiber.

Preferably, according to an embodiment, the respective light emitting section comprises exactly three loops according to an embodiment.

Further, each light emitting region on the flexible textile comprises one or more first optical fibers each comprising a light emitting section comprising preferably exactly three loops, particularly arranged in a triangular pattern, such that the multitude of light emitting sections which each light emitting region comprises is arranged in a densely fashion in order for the light emitting point to be reasonably definable as a point-like source of light.

Further, according to an embodiment, each light emitting region has dimensions similar to an LED, or 6mm x 6mm, or also 7mm x 7mm, or more preferably, 5mm x 5mm, or less.

Further, according to an embodiment of the medical device, the light receiving section of the respective second optical fiber is a curved light receiving section comprising a curvature allowing the light receiving section to collect near infrared light coming from the tissue.

According to an embodiment, the light receiving section of the respective second optical fiber comprises a loop (or is formed as a loop) for receiving near infrared light backscattered/coming from the tissue of the subject, preferably at least two loops, preferably at least three loops. Preferably, the respective light receiving section comprises exactly three loops in an embodiment. Furthermore, also the light receiving section of the respective second optical fiber can also comprise a knot, instead of a loop (e.g., one, two, three, or even more knots), or a bend, or may comprises another suitable topology allowing to collect near infrared light coming from the tissue.

Using loops further helps to achieve small localized light emitting or light receiving regions of point-like character via the proper bending radius required to get most of the light out within that small region/area, while still having enough loops to ensure proper fixation. Particularly, using three loops per light emitting section allows a secure fixation of the respective optical fiber to the textile but also allows to emit enough light via the bending losses of the loops out of a small area.

Furthermore, in an embodiment of the medical device, the flexible textile comprises supporting fibers.

Particularly, the supporting fibers form at least a portion of the inside of the garment. Particularly, the supporting fibers form a majority of the inside of the garment.

Further, in an embodiment, the respective first optical fiber is fastened to the supporting fibers. For this, particularly the respective loop of the light emitting sections is looped around at least one of the supporting fibers or otherwise integrated/fastened into the textile such as to optimize contact and light emission into, or collection out of, the tissue while remaining in place, i.e. that the coordinate of the loop or knot remain constant relative to the reference frame of the textile, i.e. that the source detector distance must remain constant while the sensor is being worn or must be determined while it changes.

Furthermore, according to an embodiment, the respective second optical fiber is fastened to the supporting fibers, too. For this, particularly, the respective loop of the light receiving sections is looped around at least one of the supporting fibers.

Furthermore, in an embodiment of the medical device, a distance between two neighboring loops of a light emitting section and/or of two neighboring loops of a light receiving section is smaller than 3mm, preferably, smaller than 1mm.

According to a further embodiment, a distance between the light emitting region comprised of one or more light emitting sections of one of the first optical fibers and the light receiving region comprised of one or more light receiving sections of one of the second optical fibers is at least 5mm, particularly at least 10 mm, particularly at least 20 mm. According to an embodiment, the distance between any given light emitting region and light receiving region between which a tissue volume of interest to be measured exists is the range from 8 mm to 60 mm.

Particularly, regarding all embodiments, the first light generating unit is preferably configured to generate and couple light having a first wavelength and light having a second wavelength into the first optical fibers, the first and the second wavelength being different from one another. The first light generating unit can also be configured to generate and couple light of a plurality of different wavelengths into the first optical fibers (such as four different wavelengths or eight different wavelengths). Furthermore, according to an embodiment, the first light generating unit can be configured to generate and couple light into the first optical fibers that comprise a broad continuous spectrum.

Furthermore, each first optical fiber is to receive light of a first wavelength from the first end and light of a second wavelength from the second end such that the light emitting section of said first optical fiber which lies between the first end and the second end is able to emit both the first wavelength and the second wavelength either simultaneously, or separated in time.

Particularly, regarding all embodiments, at least two wavelengths emitting from the same light emitting region on the textile surface is beneficial for the tissue oxygenation measurement, and the precision of said measurement increases with the number of wavelengths which can be emitted from the same light emitting region.

Particularly, in an embodiment, the first light generating unit can be configured to generate and couple light of a first wavelength, light of a second wavelength, light of a third wavelength and light of a fourth wavelength into the first optical fibers wherein the first to fourth wavelengths are different from one another.

Particularly, the respective light can comprise other wavelengths as well. Particularly, the respective light can have a spectrum comprising a maximal intensity at the respective wavelength. Particularly, using more than two different wavelengths of near infrared light allows to increase precision of the measurement.

Particularly, regarding all embodiments, the first light generating unit can be configured to generate near infrared light of a first and of (a different) second wavelength (or of even more different wavelengths, see above) and to couple near infrared light of the first and of the second wavelength into the first optical fibers. Particularly, the first light generating unit can be configured to generate and couple near infrared light of the first wavelength and near infrared light of the second wavelength successively into the respective first optical fiber.

Particularly, the control unit is configured to generate first raw data indicative of intensities of near infrared light of the first wavelength and of the second wavelength detected by the first light detector unit.

Preferably, the respective light in the above-stated examples preferably is in the range from 650 nm to 1500 nm, particularly 650 nm to 1000 nm, and can be near infrared light (NIR).

Particularly, in an embodiment, the first wavelength is in the range from 660 nm to 800 nm and/or the second wavelength in the range from 800 nm to 1000 nm, the second wavelength being different from the first wavelength. Particularly, in an embodiment, near infrared light of four different wavelengths is used, particularly: 700 nm, 740 nm, 810 nm and 880 nm. The light with the respective wavelength can be generated and coupled successively into the respective first optical fiber.

Particularly, it is also possible to use near infrared light of a broader spectrum and to differentiate between oxy-hemoglobin and deoxy-hemoglobin on the detector side.

Furthermore, in an embodiment, the first light generating unit can comprise a plurality of light sources, wherein each light source is configured to generate light, particularly said light of the first wavelength and said light of the second wavelength, wherein the respective light source can comprise a first light emitting element (such as e.g. a light emitting diode (LED)) for generating light of the first wavelength and a second light emitting element (such as e.g. an LED) for generating light of the second wavelength. It is also conceivable that the respective light source is formed by a single light emitting element that can emit the first and the second wavelength successively.

Furthermore, the first light detector unit can comprise a plurality of photodetectors, wherein the respective photodetector can be a photodiode.

Furthermore, according to an embodiment, the light emitting sections of the first fibers are arranged in groups so as to form a plurality of light emitting regions on the flexible textile, each light emitting region comprising a plurality of light emitting sections of a corresponding number of first optical fibers, wherein the first light generating unit is operatively connected to the first and second ends of the respective first optical fiber of the respective light emitting region such that each light emitting section of each first optical fiber can emit light having two different wavelengths, wherein particularly the light of the first wavelength is coupled into the first ends and light of the second wavelength is coupled into the second ends of the first optical fibers.

Furthermore, according to an embodiment, the light receiving sections of the second fibers are arranged in groups so as to form a plurality of light receiving regions of the flexible textile, each light receiving region comprising a plurality of light receiving sections of a corresponding number of second fibers, wherein photodetectors of the first light detector unit are operatively connected to the first and second ends of each of the second optical fibers of the respective light receiving region..

Furthermore, in an embodiment of the medical device, the analyzing unit is configured to determine at each point of a plurality of points in the underlying tissue a concentration of oxy-hemoglobin and a concentration of deoxy-hemoglobin in the tissue of the subject from said first raw data and to determine said oxygen saturation distribution in the tissue ($StO_2$) at each point via $StO_2 = [HbO_2]/([HbO_2] + [HHb])$, where $[HbO_2]$ denotes the concentration of oxy-hemoglobin at the respective point and $[HHb]$ denotes the concentration of deoxy-hemoglobin at the respective point.

Particularly, the equations for spatially resolved spectroscopy, multi-distance, and multi wavelength show the need for true point sources/ point detectors to do these calculations in contrast to the prior art. In the spatially resolved spectroscopy (SRS) method, the spatial derivative $\frac{\partial OD}{\partial r} = \frac{1}{\ln(10)} \left( \sqrt{3\mu_a \mu s'} + \frac{2}{r} \right)$ of the optical density OD is used. OD is defined as $OD(t, \lambda) = -\log_{10} \left( \frac{I(t,\lambda)}{I_0(t,\lambda)} \right)$. In these equations, I stands for the measured intensity, $I_0$ for the emitted intensity, r for the source-detector distance, $\lambda$ for the wavelength, t for time, $\mu_a$ for the absorption coefficient and $\mu s'$ for the reduced scattering coefficient. From this spatial derivative, a relative absorption coefficient k $\mu_a$ can be calculated:

$$k\mu_a(\lambda) = \frac{1}{3(1 - h\lambda)} \left( \ln(10) \frac{\partial OD(\lambda)}{\partial r} - \frac{2}{r} \right)^2$$, which in turn can be related to the relative hemoglobin concentrations:

$$k \begin{bmatrix} HHb \\ O_2Hb \end{bmatrix} = \begin{bmatrix} \varepsilon_{HHb,\lambda 1} & \varepsilon_{O_2Hb,\lambda 1} \\ \varepsilon_{HHb,\lambda 2} & \varepsilon_{O_2Hb,\lambda 2} \end{bmatrix}^{-1} k \begin{bmatrix} \mu_{a,\lambda 1} \\ \mu_{a,\lambda 2} \end{bmatrix}$$

, the ratio of which corresponds to the oxygen saturation in the tissue ($StO_2$).

In order to resolve changes in both oxygenated and deoxygenated haemoglobin, the solution requires at least two wavelengths. Furthermore, the SRS method requires at least two separate but parallel light paths to be measured in order to calculate the spatial derivative of the light attenuation. With two measurement points, the derivative is linear meaning the measurement points should be close enough to justify this linear assumption yet distinct enough so each point can be considered unique. This, then, requires two measurement points that each cover the smallest possible area, allowing their close proximity to not contribute to measurement error.

The spatial derivative in the SRS method then allows absolute absorption values of the tissue to be measured, given a priori scattering values. With such assumed scattering values, absolute tissue oxygen values can be calculated. Variations in the measurement geometry then probe different tissue depths, allowing the spatial derivatives and therefore absorption values to be measured at different tissue depths.

Particularly, in an embodiment, the analyzing unit is configured to issue an alert in case an oxygen saturation in the tissue is below a pre-defined threshold. Particularly, Baseline $StO_2$ is around 70-75% for the relevant tissue like muscle, and the critical tissue oxygen saturation is around 40-50%. Therefore, in an embodiment, the threshold corresponds to 50% $StO_2$, particularly 40% $StO_2$.

Furthermore, in an embodiment, the analyzing unit is configured to issue to the user an estimation of the increased risk associated with prolonged low oxygenated tissue, instead of an alarm, such that risk increases as a function of time and tissue oxygenation.

Furthermore, in an embodiment, the change in tissue oxygenation with time may be extrapolated in time to suggest to the user a point in time in the future when a critical value is reached in order to either predict a time when an alarm is issued, or to predict the risk as a function of time and tissue oxygenation.

According to a further embodiment, the medical device further comprises a plurality of third optical fibers, each third optical fiber being connected to a supporting fiber of the flexible textile, and wherein each third optical fiber comprises a first end and a second end opposite the first end, and wherein the medical device further comprises:

- a second light generating unit configured to generate and couple light into each third fiber via the respective first end,

- a second light detector unit configured to detect light (coupled into the respective third fiber) at the second end of the respective third fiber,

wherein the third optical fibers are arranged such that they form a plurality of crossover points wherein at each crossover point a third optical fiber crosses another third optical fiber so that a pressure applied to a crossover point reduces an intensity of the light detected by the second light detector unit at the second ends of the respective third fibers forming the crossover point.

Preferably, in an embodiment, the crossover points form nodes of a grid, particularly of a regular grid, particularly of a rectangular grid, particularly of a square grid.

In an embodiment, the second light generating unit is enclosed by said housing of the control unit. Furthermore, in an embodiment, the second light detector is enclosed by said housing, too.

Furthermore, the second light generating unit can comprise a plurality of light sources (each light source being e.g. an LED), each light source configured to generate and couple light into an associated third fiber at the first end of the third fiber. Furthermore, the second light detector unit can comprise a plurality of photodetectors (each photodetector being e.g. a photodiode), each light detector configured to detect light at a second end of an associated third fiber.

Further, in an embodiment, the control unit is further configured to generate second raw data indicative of intensities of light detected by the second light detector unit.

Further, in an embodiment, the control unit is configured to transmit said second raw data to the analyzing unit, particularly via a wireless communication direction or a cable, wherein the analyzing unit is configured to determine a pressure distribution assigning to each crossover point a pressure exerted onto the respective crossover point from the second raw data.

According to a further embodiment, the analyzing unit is configured to issue an alert if a pressure of said pressure distribution exceeds a predetermined threshold.

Particularly, the present invention allows both measurements, i.e. oxygen saturation and pressure, to take place concurrently, which allows the direct relation between pressure and tissue oxygenation to be measured. Furthermore, according to an embodiment of the medical device, the analyzing unit comprises a processor, particularly for executing a computer program, particularly according to the present invention.

Further, according to an embodiment, the medical device comprises a display operatively connected to the analyzing unit, the display being configured for displaying information to a user (such as said oxygen saturation, particularly oxygen saturation distribution, and/or an indication of increased risk with time, or predictive extrapolation of oxygen saturation in time, and/or specifically spatially resolved tissue oxygenation, and/or said pressure distribution). Particularly, in an embodiment, the analyzing unit can be a computer such as desktop computer connected to a display.

Furthermore, in an embodiment, the analyzing unit can be portable unit, particularly a handheld unit.

Particularly, in an embodiment, the analyzing unit can comprise the display. Particularly the analyzing unit can be a laptop, a tablet computer or a mobile phone such as a smart phone comprising the processor and the display, particularly comprising a touch screen.

In an embodiment, the respective first optical fiber and/or the respective second optical fiber and/or the respective third optical fiber comprises a polymer or consists of a polymer. According to a further aspect of the present invention, a method for monitoring oxygen saturation in a tissue using a medical device according to the present invention is defined in claim 15.

Generally, in all embodiments, the light is preferably light having a wavelength in the range from 650 nm to 1500 nm, particularly 650 nm to 1000 nm. Particularly said light is near infrared light.

Particularly, according to a preferred embodiment, step iii) corresponds to determining with the analyzing unit for each of a plurality of points in the tissue a concentration of oxy-hemoglobin ($[HbO_2]$) and a concentration of deoxy-hemoglobin ($[HHb]$) in the tissue from the first raw data, wherein an oxygen saturation value ($StO_2$) at each point is determined as $StO_2 = [HbO_2]/([HbO_2] + [HHb])$, so as to provide an oxygen saturation distribution assigning to each of said points an oxygen saturation value of the tissue, where $[HbO_2]$ denotes the concentration of oxy-hemoglobin at the respective point and $[HHb]$ denotes the concentration of deoxy-hemoglobin at the respective point, wherein particularly these points are uniquely measured in all three dimensions

Preferably, in an embodiment, the respective light emitting section is oriented and arranged such that near infrared light is emitted into the surface of the tissue in a direction comprising a component parallel to a surface normal to the tissue, wherein said direction is ideally parallel to said surface normal, and/or wherein the respective light receiving section is oriented and arranged such that an optical axis of near infrared light collection of the respective light receiving section comprises a component parallel is parallel to a surface normal of the tissue from which near infrared light is being collected, wherein said optical axis is ideally parallel to said surface normal of the tissue from which near infrared light is being collected.

According to an embodiment of the method, step ii) further comprises coupling light into each third optical fiber at its first end and detecting light coupled into the respective third fiber at the second end of the respective third fiber with the second light detector unit.

Furthermore, in an embodiment of the method, step ii) further comprises generating second raw data indicative of intensities of light detected by the second light detector unit, and transmitting said second raw data to the analyzing unit, particularly via a wireless communication direction or a cable.

Furthermore, in an embodiment of the method, step iii) further comprises determining with the analyzing unit a pressure distribution from the second raw data, the pressure distribution assigning to each crossover point a pressure exerted onto the respective crossover point. According to a further embodiment of the method, the normal oxygen saturation distribution is determined for the specific subject when the pressure exerted onto the respective crossover point is zero over a predetermined time span.

According to an embodiment of the method, the method further comprises the step (iv) of issuing an alert (e.g. via the display of the medical device) if an oxygen saturation value at a point in the tissue is below 50%, particularly below 49%,and/or issuing an alert if a pressure determined by the analyzing unit is above a predefined threshold.

In the following, embodiments of the present invention as well further features and advantages and other aspects of the present invention shall be described with reference to the Figures, wherein

Fig. 1    shows tissue oxygen saturation as a function of time due to applied external pressure, a) shows unrestricted blood flow and normally-oxygenated tissue in the absence of external pressure being exerted on the tissue, b) shows a steady decrease in StO2 with time due to compression of the tissue and restricted blood flow due to constantly applied external pressure, c) shows the onset of necrosis due to critically low StO2, d) the onset of irreversible damage due to tissue necrosis as a result of prolonged critically low StO2 in the tissue happening as quickly as 1 hour after critically low StO2 is reached,

Fig. 2    shows the most common pressure points which can cause pressure injuries,

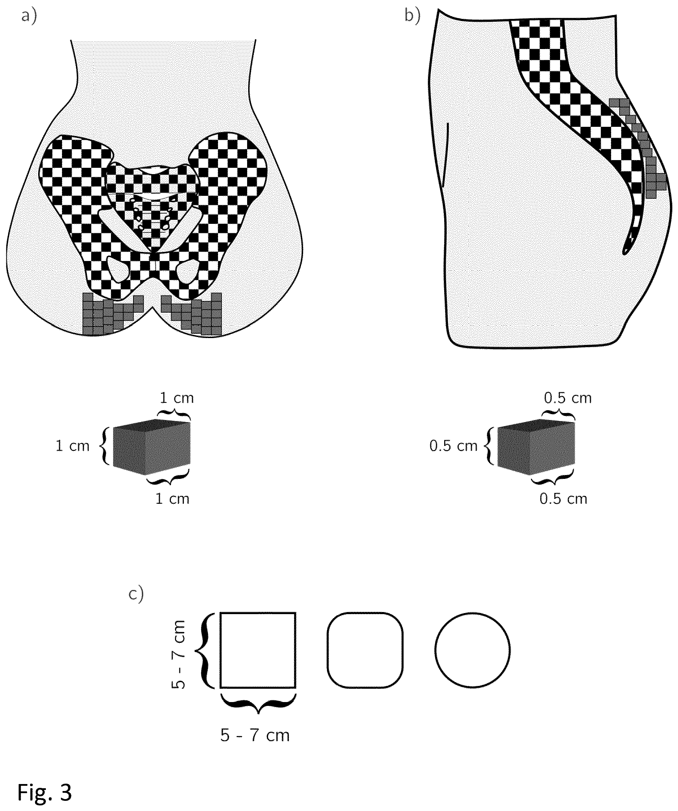

Fig. 3    shows susceptible PI regions and measurement regions of interest in the tissue; particularly, the region under the ischial tuberosity (a) and is much thicker than that of the sacrum (b). The resolution therefore needs to be higher in the sacrum region to measure the thinner tissue. The region below the sit bone is much thicker and will often contain high adipose tissue thickness (ATT), however, ATT should not matter as it is expected that the StO2 is the same in all tissues; c) shows different possible shapes of the sensor region and the expected size of the region of interest,

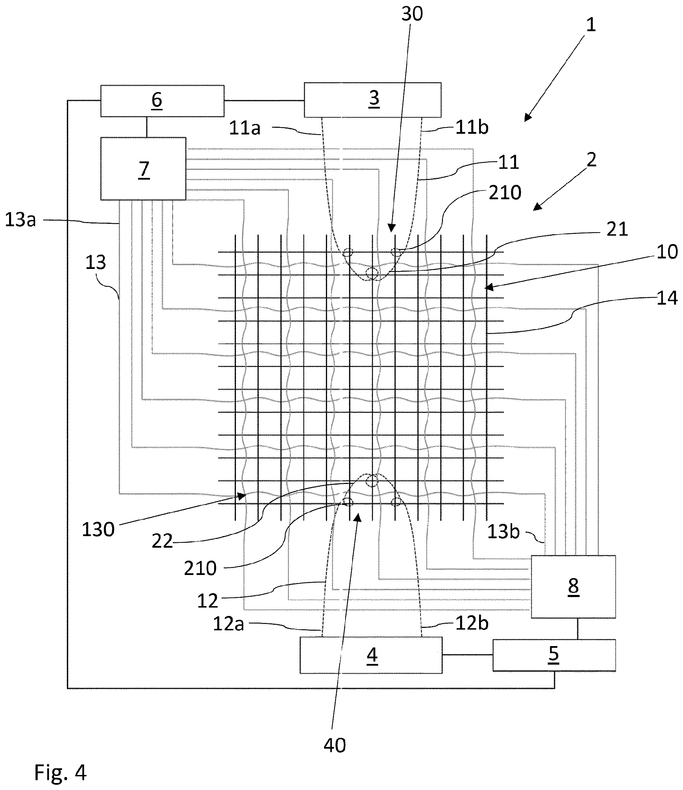

Fig. 4    shows an embodiment of a medical device according to the present invention,

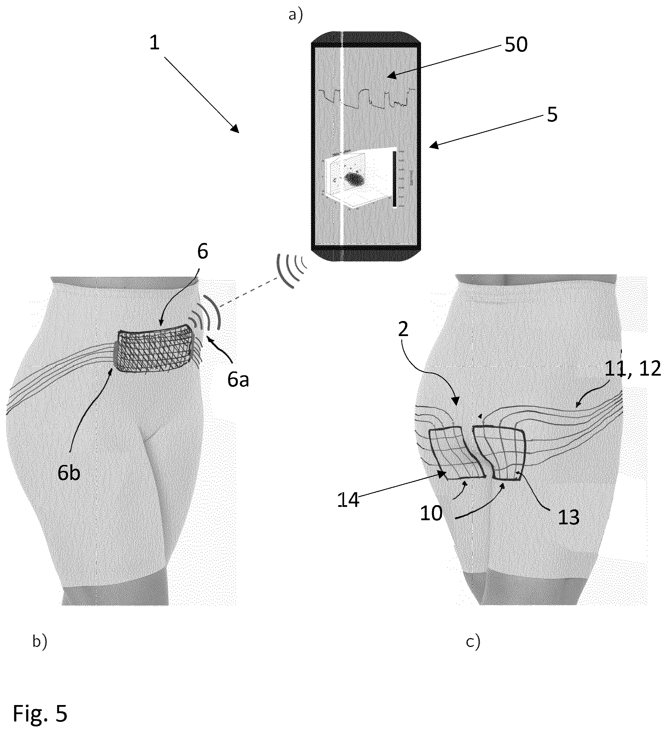

Fig. 5    shows an example of the location of the flexible textile of the medical device, particularly forming textile integrated sensor, within a pair of elastic shorts. a) shows the wirelessly-connected analyzing unit, b) shows the control unit connected to optical fibers which lead to the sensing area, c) shows the textile-integrated sensor located at the ischial tuberosity region connected to fibers leading to the control unit,

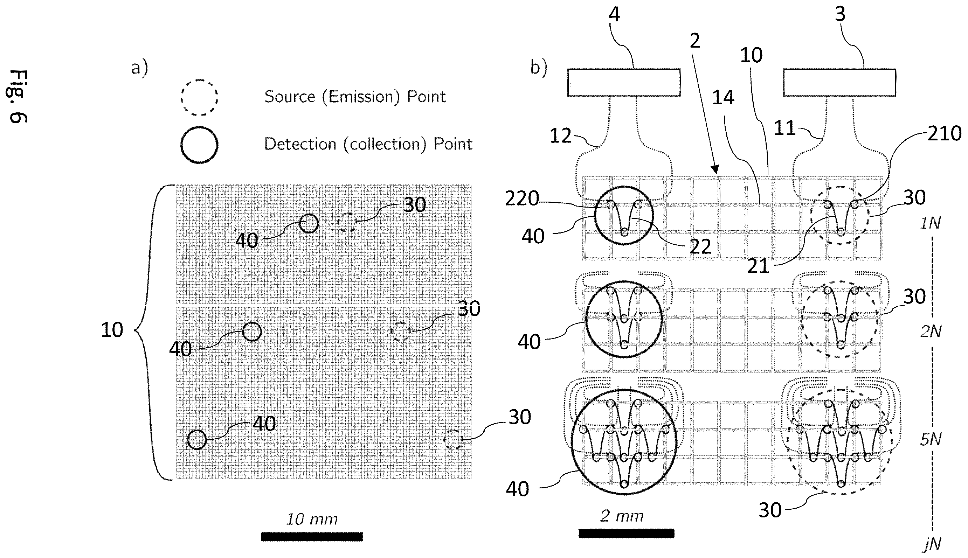

Fig. 6    shows an embodiment of the flexible textile of the medical device according to an embodiment of the invention, wherein various possible stitching profiles for optical fiber (e.g. POF) textile integration are indicated. Particularly, emission points and detection points may be close together or far apart, depending on the desired measurement depth into the tissue (a). Farther apart (a greater SDD) corresponds to greater measurement depth. Each optical fiber (POF) is stitched into the textile using 3 loops to ensure proper fixation (b). To guarantee that enough light is emitted or collected at larger distances (when the signal is weaker) multiple POFs can be densely stitched into an area. Therefore, emission points and collection points are often small groups of POFs, each integrated into the textile with 3 loops and connected to a common light source or light detector. j = the number of POFs present in each emission points or collection point, and N = 3 is the number of loops per POF. therefore, 5N corresponds to an emission point or collection points with 15 loops. The number of free fiber ends to be connected to either a light source or a light detector is 2j. Therefore, 5N corresponds to 10 free fibers ends. j may be any number of 3-loop optical fibers,

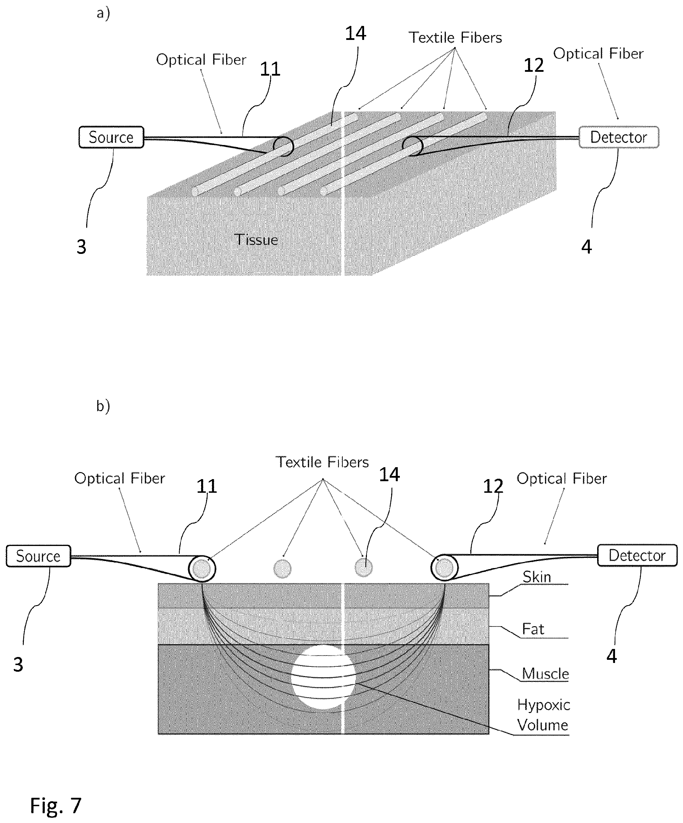

Fig. 7    a) shows optical fibers (e.g., POFs) fixed onto the textile host formed from supporting fibers for the measurement of tissue oxygen saturation via NIRS, b) shows a specific source-detector distance (SDD) and a corresponding measurement depth. Particularly, the heavier and denser the curved lines between the source and detector point (the light path) the more sensitive the device is to changes in the optical properties of the tissue at that location,

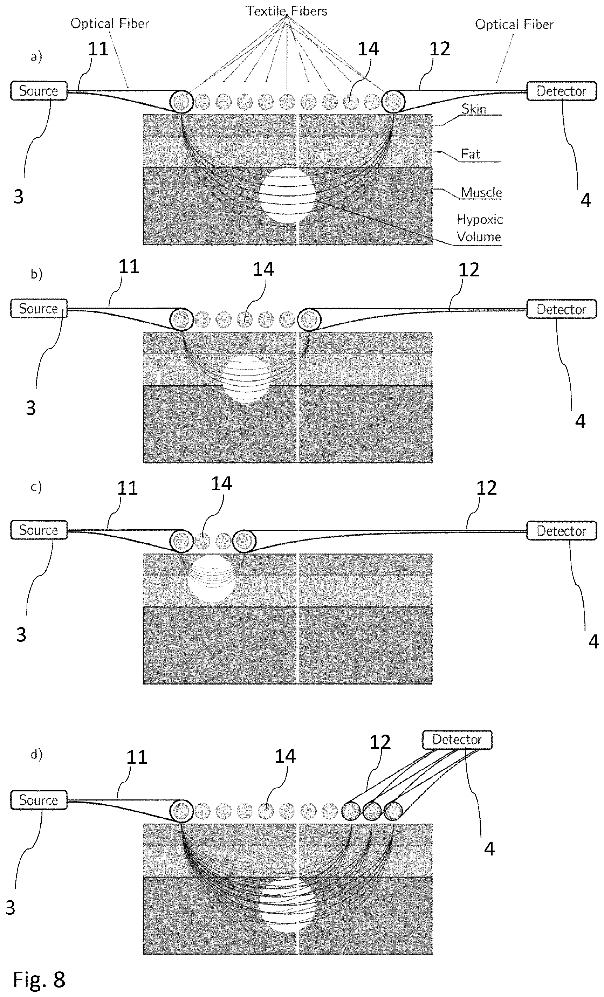

Fig. 8    (a-c) shows that different source-detector distances (SDD) measure into different tissue depths, wherein d) shows closely grouped loops of optical fibers can act as a single detector capable of measuring a similar tissue depth with greater sensitivity,

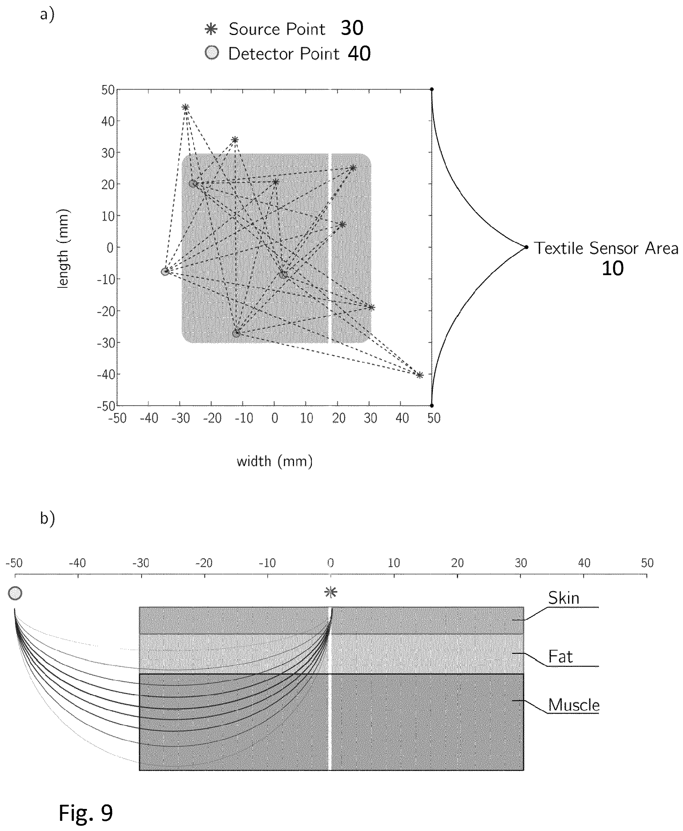

Fig. 9     a) shows an example of a top view of an optimized source-detector geometry. Particularly, the patch size is preferably greater than the measurement area (as shown in b)). This is because any given combination of a source and detector will detect changes in tissue physiology up to a maximum measurement depth in the tissue located at the midpoint between the source and detection point. The thin, curved lines in the side view (b) illustrate the light path through the medium. The denser and heavier the lines, the more sensitive to changes in chromophore concentration that region is. To measure at the boundaries of the measurement region of interest, the sources and detectors muss lie across the boundaries of this region. The geometry must measure through different tissue layers and resolve different hypoxic volumes at different degrees of hypoxia and depth and size. Therefore, an optimized geometry combines long and short SDDs to measure physiological changes in both shallow and deep tissue. The unintuitive geometry resulting from optimization is due to constraints on the maximum and minimum SDD and the maximum and minimum number of source points and detection points. These values are defined by practical limitations,

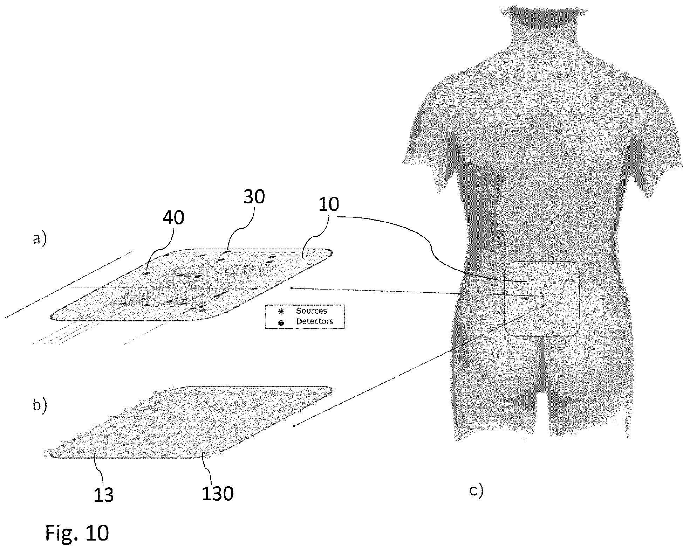

Fig. 10     shows an example of the source-detector pattern integrated onto a textile host garment and placed at the critical region of the sacrum,

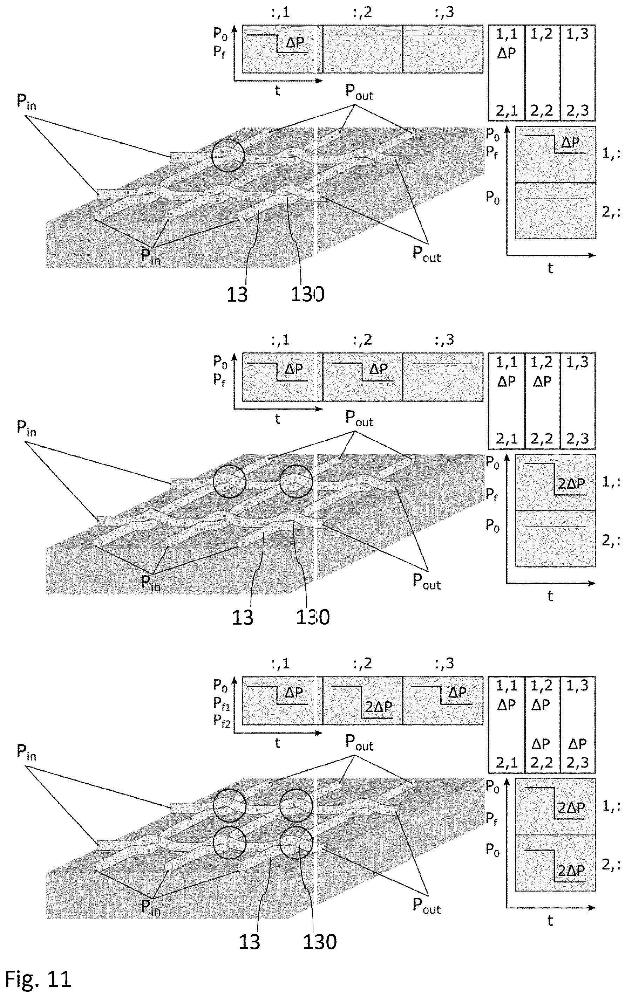

Fig. 11     shows a photonic pressure sensor according to an embodiment of the present invention. Particularly, the photonic pressure sensor uses crossover points to generate pressure and compression of the light-transmitting optical fibers (e.g. POFS). The compression of the fibers at these points results in a change of light intensity measured from the end of the fiber opposite of the end at which light is transmitted into it. These changes can be used to calculate a pressure map across the region of the sensor.

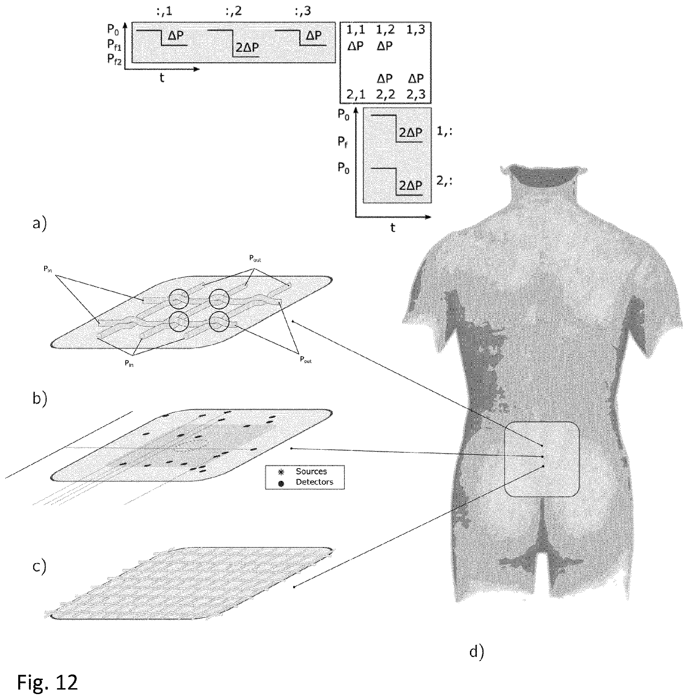

Fig. 12     shows an example of the source-detector NIRS pattern integrated together with the photonic pressure sensor onto a textile host garment and placed at the critical region of the sacrum.

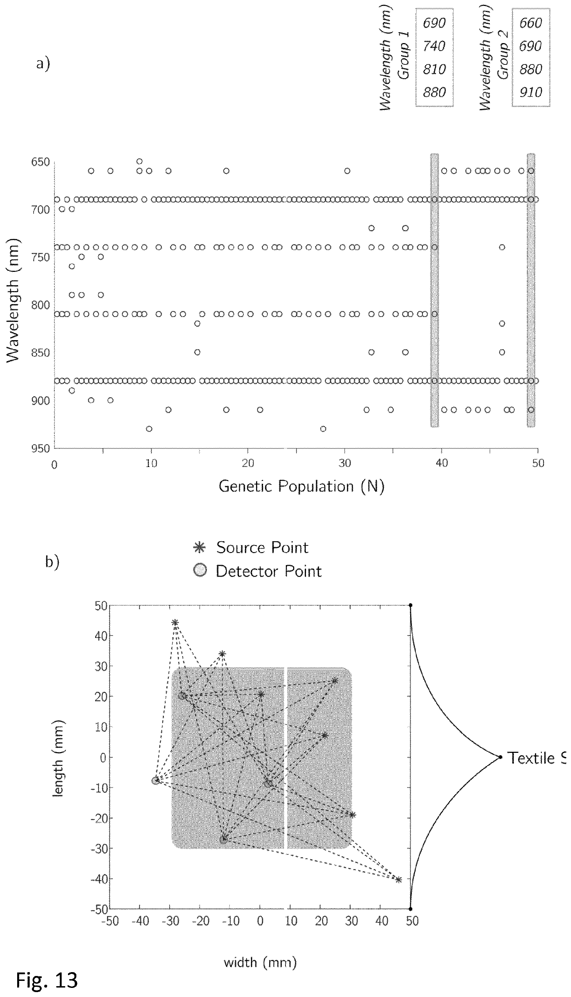

Fig. 13     a) and b) show an example of an optimized wavelength group and source-detector geometry as a result of a genetic algorithm, respectively. Particularly, two groups resulted from the genetic algorithm which tried to find a solution which optimized the physiological measurement of tissue oxygen saturation in tissue with both thin and thick fat layer (ATT). Therefore, the two competing solutions are likely optimized for tissue with low fat thickness and tissue for greater fat thickness, respectively. The example geometry may also change if specialized,

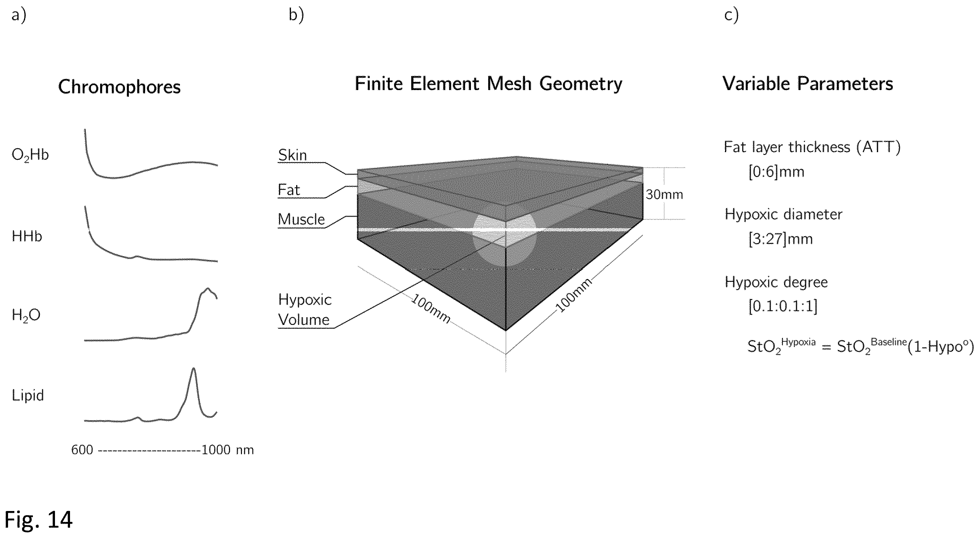

Fig. 14     b) shows a tissue composition used in the finite element forward calculation and reconstruction method, c) shows ranges of parameters and a) shows relative chromophore absorption spectra,

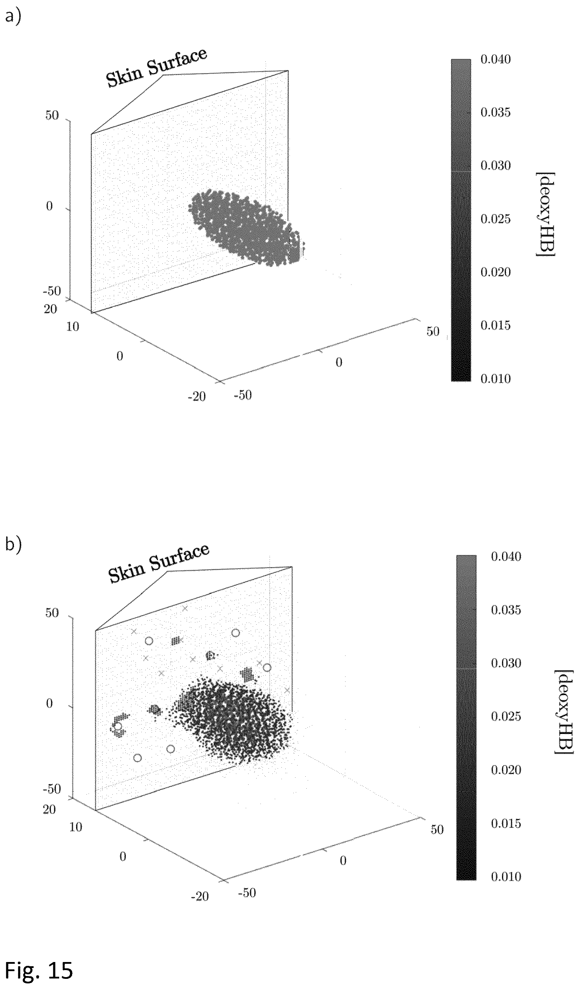

Fig. 15     shows results of the reconstruction of the concentration of deoxy-hemoglobin [HHb]. a) True, b) reconstructed. The x points mark sources and the circles detectors on the skin's surface, and

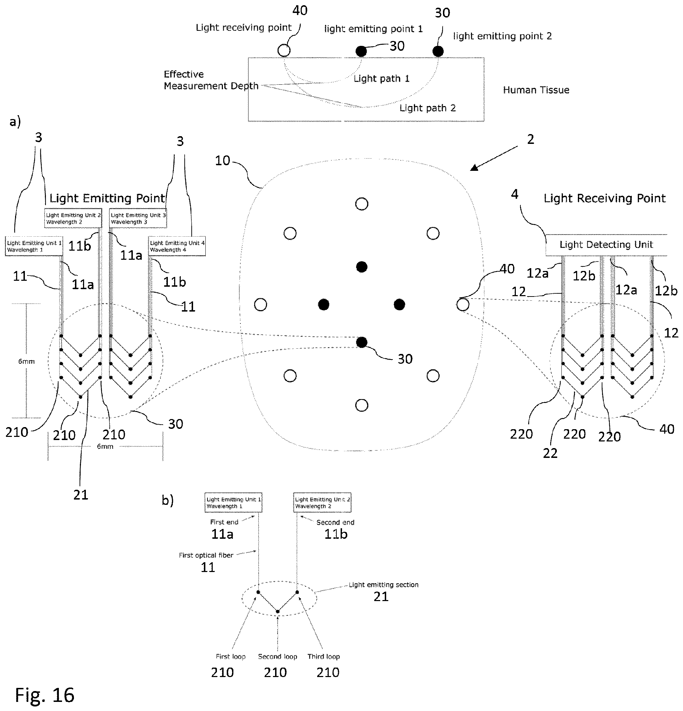

Fig. 16     shows a further preferred embodiment of a medical device according to the present invention.

In order to overcome the problem of a subject being unaware of the critically low oxygenated regions in their tissue resulting from prolonged sitting or lying down or being positioned in any way for a duration long enough to allow pressure injuries to develop, the present invention provides a medical device 1 which allows the physiological changes in the tissue to be measured without creating additional pressure points by integrating light emission points 30 and light collection points 40 (also denoted as light emitting regions 30 and light receiving regions 40) in an arrangement optimized for spatially resolved $StO_2$ measurements using multiple wavelengths emitted from localized light emitting regions 30 into a wearable textile 10 or garment which are on the order of the textile's fiber diameter and stiffness to avoid the presence of additional pressure points. The emission points 30 and collection points 40 emit and collect, in the case of detecting the chromophores oxy-hemoglobin and deoxy-hemoglobin, suitable light, particularly near-infrared light. The source 3 of the light may be the emission points 30 themselves or may be coupled in remotely. The collection points 40 may couple the collected light to a remote light detector 4, or the collection points 4 may be light detectors themselves. All remote components such as data processors (e.g., control unit 6), light sources 3, light detectors 4 and power sources are housed in a compact unit able to be comfortably carried by the user. This allows the entire device to be truly portable. This compact unit may also be integrated into or mounted onto the textile as long as it does not introduce pressure points. A preferred embodiment of the present invention is schematically depicted in Fig. 4.

To further enhance the ability of the sensor 2 to not only prevent, but to better understand the formation of pressure injuries and to help calibrate and correct for movements and changes in tissue geometry, a photonic pressure sensor can be integrated into the host textile concentric and co-planar to the $StO_2$ sensor. This also allows the device 1 to be used as a clinical research tool. The relation between pressure at the surface and pressure injuries within the tissue is not exactly known. The additional integration of a pressure sensor (e.g., based on third optical fibers 13 forming crossing points 130) can help to uncover the nature of this relation to better understand under what circumstances pressure injuries occur. The integration of a pressure sensor also allows one to account for changes in the optical signal due to the level of contact between the textile and the user. It also allows the optical device to calibrate itself by sensing when the user has moved into a position which does not apply pressure to the part of the body being monitored. When the pressure sensor reads no pressure, the oxygenation sensor can determine the baseline tissue oxygenation for the specific subject. Furthermore, changes in tissue geometry and thickness of the tissue layers can be corrected for when the pressure field in the region of the $StO_2$ sensor is known.

The selection of the proper materials is important when considering a wearable sensor 2, especially when it should be worn for a long duration, needs to stay in place, should reduce moisture and remain comfortable. The host textile 10 into which the sensor 2, particularly NIRS tissue oxygenation sensor, and the photonic pressure sensor are integrated needs to meet these demands.

The means by which the light, particularly near-infrared light (see also above regarding suitable wavelengths) is emitted into the skin and collected from the skin at a different location at a known distance from the emission point is through the use of optical fibers 11, 12 which are tightly stitched or embroidered or otherwise effectively integrated into the host textile 10. Each emission point 30 and collection point 40 is composed of a tightly arranged group of light emitting sections 21 and light receiving sections 22, which can comprise tightly curved sections such as e.g., loops 210, 220, knots or other bend structures. The higher the local curvature of the optical fibers 12, 13 at theses sections (e.g., loops 210, 220), the more light can be transmitted out of them and into the tissue and, likewise, the more light can be captured back into the respective optical fiber from the tissue [2]. Because the amount of emitted light or collected light depends on the number of loops 210, 220 (or other tightly-curved segments of the respective optical fiber) that make up each emission or collection point 30, 40, multiple loops 210, 220 (or knots or adequately tightly-curved segments) anywhere from 3 to more than 20, may be needed at each point. This requires optical fibers 11, 12 that are thin and flexible enough to be bent to a small enough radius to match the textile supporting fibers 14 of the host textile 10. The optical fibers 11, 12 should also have very low light attenuation to ensure that the (e.g., infrared) light sources 3 and detectors 4 work as efficiently as possible; necessary for the portability of the device 1. The optical fibers 11, 12 should also be soft enough to not produce rigid pressure points against the tissue. Furthermore, they should be bio-compatible and washable. The optical fibers 11, 12 should preferably also possess mechanical properties allowing the optical fibers 11, 12 to be integrated into the host textile 10 with commercial machinery without breaking. Preferably, optical fibers belonging to the class called polymer optical fibers (POF) are used in the context of the present invention.

Fig. 16 shows a further preferred embodiment of the present invention. According thereto, the sensor 2 comprises a flexible textile 10 comprising a plurality of first optical fibers 11 and a plurality of second optical fibers 12, wherein the first optical and second optical fibers 11, 12 each comprise a first end 11a, 12a and a second end 11b, 12b.

The sensor 2 further comprises a first light generating unit 3 configured to generate and couple light of a first wavelength into the first ends 11a of the first optical fibers 11 and to generate and couple light of a second wavelength into the second ends 12a of the first optical fibers 11, the first wavelength being different from the second wavelength. This configuration is shown in Fig. 16 b). However alternatively, as shown in Fig. 16 a), further first optical fibers 11 can be used in an analogous fashion, to couple light of a third wavelength into first ends 11a of further first optical fibers 11 and light of a different fourth wavelength into the second ends 11b of the further first optical fibers 11. Particularly, the first, second, third and fourth wavelength are different from one another.

Furthermore, as indicated in Fig. 16, the respective first optical fiber 11 is configured to emit light via a light emitting section 21 of the respective first optical fiber 11 to irradiate tissue of the subject, the light emitting section 21 being arranged between the first end 11a and the second end 11b of the respective first optical fiber 11, and wherein the respective second optical fiber 12 comprises a light receiving section 22 configured to receive light coming from the tissue, the light receiving section 22 being arranged between the first end 12a and the second end 12b of the respective second optical fiber 12.

Furthermore, the sensor 2 further comprises a first light detector unit 4 operatively connected to the second optical fibers 12 and configured to detect light received by the light receiving section 22 of the respective second optical fiber 12.

Further, an analyzing unit can be used to determine an oxygen saturation value of the tissue using intensities of light detected by the first light detector unit 4 as e.g. described herein.

Furthermore, preferably, the light emitting sections 21 of the first fibers 11 are arranged to form a plurality of separate light emitting regions 30 on the flexible textile 10, each light emitting region 30 comprising a plurality of light emitting sections 21 of a corresponding a number of first fibers 11. The light emitting regions can have a diameter of about 6mm according to an embodiment. Further, the light receiving sections 22 of the second fibers 12 are arranged to form a plurality of separate light receiving regions on 40 the flexible textile 10, each light receiving region 40 comprising a plurality of light receiving sections 22 of a corresponding number of second fibers 12.

**EP 4 340 721 B1**

Preferably, the light emitting section 21 of the respective first optical fiber 11 comprises at least three loops 210, which are preferably arranged in a triangular configuration, i.e., the loops 210 are arranged at locations, which locations define the corners of a virtual triangle.

In the same fashion, the light receiving section 22 of the respective second optical fiber 12 comprises at least three loops 220, which are preferably arranged in a triangular configuration, too.

Tissue oxygen saturation is measured at regions prone to the development of pressure ulcers. For paraplegics, the most common areas are the sit bone (ischial tuberosity) and the tail bone of the lower back (sacrum). The former region is susceptible due to the pointed shape of the bones which bare the full weight of the person in the seated position and which press against layers of muscle, fat and skin. The latter region is susceptible due to the pointed shape of the sacrum and the very thin tissue between it and the skin (cf. Fig. 3). In order to monitor these regions, the textile-integrated sensor 2 can be located at both sit bones and the sacrum. Corresponding sensors can all be integrated into the same piece of clothing, such as cycling shorts. The total measurement area of each region can be around 7cm x 7cm according to an embodiment (but may also be larger or smaller depending on the respective application). Particularly, this size allows one to measure deeply into the tissue at the bone/muscle interface, a task for which large source-detector separations are beneficial. Resolving changes in oxygenation at this depth (and not just on average within the tissue including changes at this depth) requires multiple light emitting regions emitting multiple wavelengths and multiple light receiving regions arranged in an optimized way.

Further, the voxel size (volumetric spatial resolution) can be 1cm3 for the sit bone (ischial tuberosity) and 0.5cm3 for the sacrum region, respectively (cf. Fig. 3). The actual sensor can e.g., comprise a dimension of 10cm x 10cm because the sources and detectors are preferably located outside of the measurement boundaries (cf. Fig. 9). Particularly, according to an embodiment, the sensor 2 is as a textile- integrated sensor 2 in which the sensor 2 is directly integrated into a clothing or garment which can be washed. Alternatively, the sensor 2 can be an adhesive patch (such as 10cmx10cm), e.g., for single-use applications.

The basic principle of NIRS is to shine light of a certain near-infrared wavelength into the tissue at the surface of the skin at a certain location. The light then scatters and absorbs within the tissue depending on the size of scattering elements and the absorption potential of the chromophores. A portion of the incident light is scattered back to the surface. A photo sensor 4 is placed at the surface of the skin at some distance from the light source 3 and the photon flux is measured. This measurement is repeated for different source-detector distances (SDD) and at two or more wavelengths. Small SDD measure the light scattered by tissue near the surface, while large SDD measure the light scattered by deeper tissue (Fig. 8). In the case of frequency domain NIRS (FD-NIRS), the phase of the detected light (due to the delay of the signal traveling through tissue a distance larger than the direct distance between the source and detector) can be measured as well, which allows for the determination of the absolute values of the optical properties. By using the diffusion approximation to the radiative transport equation, the absorption coefficient $\mu a$ and reduced scattering coefficient $\mu s'$ can be calculated for each wavelength, for each SDD (tissue depth). Then, the modified Beer-Lambert law (MBLL) can be used to calculate the concentrations of the chromophores. The chromophores of interest when measuring the tissue oxygen saturation are oxy-hemoglobin and deoxy-hemoglobin. The concentration of oxy-hemoglobin is denoted $[HbO_2]$ while the concentration of deoxy-hemoglobin is denoted $[HHb]$. The blood of normally oxygenated tissue typically has a tissue oxygen saturation $(StO_2)$ of 70-70% where $StO_2 = [HbO_2]/HbT$ where $HbT = [HbO_2] + [HHb]$ is the total hemoglobin. By measuring the changes in the concentrations of these two chromophores, the $StO_2$ can be instantaneously measured.

The present invention takes into account two additional chromophores: $H_2O$ and lipid to account for water content and the fat layer between skin and muscle by using four wavelengths. When the number of chromophores is equal to the number of wavelengths, the MBLL can be solved exactly (this requires that the number of wavelengths used matches the number of chromophores, and further requires that the light with the different wavelengths is emitted from the same light emitting regions, which the present invention is particularly designed for. The fat layer is expected to be an important factor due to paraplegics who develop high degrees of adipose tissue thickness (ATT) in the regions of interest. The MBLL can be written as

$$\begin{bmatrix} \Delta[HHb] \\ \Delta[O_2Hb] \end{bmatrix} = \frac{1}{r}\begin{bmatrix} \varepsilon_{HHb,\lambda1} & \varepsilon_{O_2Hb,\lambda1} \\ \varepsilon_{HHb,\lambda2} & \varepsilon_{O_2Hb,\lambda2} \end{bmatrix}^{-1}\begin{bmatrix} \Delta OD_{,\lambda1} \\ \Delta OD_{,\lambda2} \end{bmatrix}$$

when two wavelengths are considered or as

$$\begin{bmatrix} \Delta[HHb] \\ \Delta[O_2Hb] \\ \Delta[H_2O] \\ \Delta[Lipid] \end{bmatrix} = \frac{1}{r}\begin{bmatrix} \varepsilon_{HHb,\lambda1} & \varepsilon_{O_2Hb,\lambda1} & \varepsilon_{H2O,\lambda1} & \varepsilon_{Lip,\lambda1} \\ \varepsilon_{HHb,\lambda2} & \varepsilon_{O_2Hb,\lambda2} & \varepsilon_{H2O,\lambda2} & \varepsilon_{Lip,\lambda2} \\ \varepsilon_{HHb,\lambda3} & \varepsilon_{O_2Hb,\lambda3} & \varepsilon_{H2O,\lambda3} & \varepsilon_{Lip,\lambda3} \\ \varepsilon_{HHb,\lambda4} & \varepsilon_{O_2Hb,\lambda4} & \varepsilon_{H2O,\lambda4} & \varepsilon_{Lip,\lambda4} \end{bmatrix}^{-1}\begin{bmatrix} \Delta OD_{,\lambda1}/DPF_{,\lambda1} \\ \Delta OD_{,\lambda2}/DPF_{,\lambda2} \\ \Delta OD_{,\lambda3}/DPF_{,\lambda3} \\ \Delta OD_{,\lambda4}/DPF_{,\lambda4} \end{bmatrix}$$

12

when four wavelengths are considered, allowing for an exact solution where r is the SDD and $\varepsilon$ is the molar extinction coefficient for each chromophore at each wavelength. The differential path length factor (DPF) is a wavelength-dependent constant which adjusts the SDD so that the mean pathlength of the light through the tissue is considered instead.

$$\Delta OD(t, \lambda) = -\log_{10} \left( \frac{I(t,\lambda)}{I_0(\lambda)} \right)$$ and describes the change in optical density by comparing the measured light intensity before a change in chromophore concentration to the measured light intensity after a change in chromophore concentration. Increasing the number of wavelengths increases the accuracy of the calculation.

The present invention's approach to measuring StO2 is based on arranging the optical fibers (particularly POFs) into the textile in a way which allows for optimized geometries of discrete POF-based emission and detection points which can be described as true point-like sources and point-like detectors in such a way that spatial resolution spectroscopy using 2 or more wavelengths becomes possible. The approach of the present invention therefore reduces the number of fiber and fiber loops in contact with the skin, and decrease the area over which a light emitting region exists in the textile, which improves flexibility of the textile and most importantly allows for higher precision of oxygen saturation calculation in all three spatial dimensions by allowing multiple wavelengths to be emitting from a single emission point and to be detected by a single detection point. Light is transmitted into the source fibers from a small, compact unit containing a power supply and several LEDs. The light is transmitted up until the point at which the POF is integrated into the host textile and then leaves the fiber due to a high degree of local curvature of the POF. Because the POF is tightly stitched in the shape of a loop around the host textile fibers, the fiber is highly curved at the point where it is fastened. This curvature allows the light to be transmitted from the fiber instead of internally via total internal reflection. The light then enters the skin and the underlying tissue. The light is recovered by identically integrated POFs at different distances from the source using the same principle; light enters the POF at points of high curvature and is then transmitted via TIR to a light detector located within the same compact unit which houses the LED sources (Fig. 7 and Fig. 5). The light sources are time multiplexed such that each detection point measures the light from each source point, for each wavelength, separately.

Each source point 30 and detection point 40 in the textile 10 is, in reality, not just a single stitched loop of POF; each point is a collection of loops 210, 220 stitched as densely as possible. The first reason is because the efficiency of transmitting and collecting light into and out of fibers through the method of bending loss is less efficient than placing a cut and polished end of an optical fiber directly against the tissue parallel to the tissue surface normal. In order to measure the light from deep regions of tissue where PI form, a large fluence rate (photon density) is required. Furthermore, for the optical fibers (e.g., POF) 11, 12 to remain well-attached to the host textile 10, 3 or more loops 210, 220 are required. At the same time, light is lost during each loop, so the number of loops per optical fiber should be small. The invention preferably uses three loops 210, 220 per optical fiber 11, 12, and multiple optical fibers 11, 12, to form each source point 30 or detection point 40 (Fig. 6). In classical NIRS, each source point 30 and each detection point 40 are ideally point sources and point detectors. This is due to the diffusion equation which assumes a precise SDD. The greater the effective surface area is of a collection of emission or detection loops/knots/etc., the greater the contribution to the measurement error due to an imprecisely-defined SDD. In addition, because multiple wavelengths are required and the SDD for each wavelength should be identical, an intrinsic error can be avoided when each point-like source (light emitting region) can emit light of multiple wavelengths from exactly the same region.

Therefore, the individual loops 210, 220 that make up each source point (light emitting region) 30 and detection point (light receiving region) 40 are as densely stitched as possible. Further, the first end of each first optical fiber and second end of the same first optical fiber are each illuminated with a different wavelength such that each light emitting section emits multiple wavelengths. Further, multiple SDD are required to measure different tissue depths. The geometry and number of the source points 30 and detection points 40 should be optimized to reduce the number of points in order to reduce the complexity of the electronics, cost of manufacturing, and time needed for analyzing the data. This optimization likewise is done to increase spatial resolution and depth resolution. Such optimized configurations were performed and different geometries calculated for subjects of various ATT and for sensors placed at various regions (Fig. 9 and Fig. 10).

The textile-integrated pressure sensor is a photonic sensor which is woven into the host textile 10 and covers the same area of the tissue surface as the (e.g., NIRS) sensor 2. Light is coupled into the optical fibers 13 at one end 13a and is detected at the other end 13b. The sources 7 and detectors 8 are preferably housed in the same compact, wearable unit as the electronics (particularly control unit) for the sensor 2. The optical fibers 13 are arranged in the host textile 10 in an orthogonal grid pattern. Each crossover point 130 acts as a measuring point, in that applying pressure to the sensor generates pressure at each crossover point 130. The pressure compresses the optical fibers 13 locally at each crossover point 130, reducing the intensity of the light which is detected from the fiber end 13b opposite of the light source 7. By time multiplexing, the pressure at each crossover point (130) can be calculated and a pressure field can be interpolated for the entire measurement region (Fig. 11). The sensor 2 and the photonic pressure sensor based on the third optical fibers 13 are integrated into the same textile garment 10 at the same location. Neither creates additional pressure points against the skin, and therefore enables real-time pressure and StO$_2$ measurements. The combination of the pressure readings also allows the NIRS signal to be corrected for changes in tissue geometry and movement of the sensor 2. Furthermore, it allows the NIRS sensor 2 to self-calibrate whenever the subject is moved to a position where there is no pressure being applied to the region of interest. Because blood flow, and hence tissue oxygenation, recover quickly (often within one minute) a zero-pressure signal can be used to determine a baseline $StO_2$ value to avoid signal drift.

The process of determining the optimal wavelengths of the light sources 3 as well as the SDD geometry and number of source points 30 and detector points 40 needed to best recover $StO_2$ in tissue with various fat and muscle thicknesses while minimizing the number of source points 30 and detector point 40 was complex. First, a wide range of tissue geometries, each with a different ATT and muscle thickness are created in the form of finite element meshes and different chromophore concentration assigned to different layers. Then a hypoxic volume is placed in the tissue mesh at different depths, with different sizes and different degree of hypoxia (Fig. 14). Each configuration is then assigned a randomly placed source point 30 and randomly placed detection point 40 and two wavelengths. The forward calculation is done using finite element analysis (FEA) and the light intensity at each detection point 30, at each wavelength, is calculated. From this, the chromophore concentrations are reconstructed for the entire volume. The reconstructed mesh is compared against the true mesh using the L2 norm (least squares method). Then, through an optimization method based on stochastic gradient descent with momentum, the geometry and number of source points 30 and detector points 40 as well as the wavelengths and number of wavelengths are changed after each iteration and the aforementioned forward calculation and fitness (L2) calculation repeated. The convergence is bound by factors such as minimal and maximal SDD, number of wavelengths, the spectrum of wavelengths, signal strength, and number of detector points 40 and source points 30. These limitations are based on hardware limitation and other physical constraints. After each tissue configuration has an optimized patch design associated with it, the results are fed into a genetic algorithm which seeks to further optimized these configurations to find a general solution for all tissue thicknesses and hypoxic geometries. These geometries and wavelength configurations have been constructed and experiments have been successfully conducted against silicon phantoms in the lab.

Silicon phantoms with absorption and scattering properties analogous to human tissue (muscle and fat) are used to test the hardware and results of the optimization. Phantoms are also made with hypoxic volumes embedded in the center to be able to determine the degree to which such anomalies can be detected. The measurements are done both with an industry standard FDNIRS device (ISS Imagent) and using the medical device as disclosed herein. The first textile patches with different test geometries of source points and detector points are soon being testing on phantoms in order to select the computed geometries which perform the best in the lab.

The genetic algorithm reached a solution (two populations which had the most common groupings of wavelengths) after about 50 generations. Each of the two groups seems to be optimized for tissue compositions with either thin or thick ATT. Multiple source-detector geometries were also found to exist, likely also for different extremes of possible tissue compositions (Fig.13). Reconstruction tends to show a good agreement to the true tissue composition, with accuracy dropping more quickly with thicker ATT than with decreased size of hypoxic volume (Fig. 15).

One particular embodiment of the textile NIRS sensor for preventing pressure injuries is having the textile integrated light emission points 30 and light collection points 40 integrated into a comfortable pair of snug-fitting under wear, such as athletic underwear or cycling shorts. These types of materials have already been developed for prolonged wear, comfort, moisture wicking and a consistent fit; all properties advantageous to the measurement of tissue oxygenation by the use of integrated fiber optics. Because a sensor is needed at the lower back (Sacrum) special shorts may be tailored to have a high waist band to allow good contact at the lower back. These types of underwear are frequently used in cycling. Another embodiment of the textile NIRS sensor for preventing pressure injuries is the placement of the light emitters and light collectors in a pair of socks. Yet another embodiment of the invention is a bed sheet, pillow case, or seat cushion cover with the textile integrated NIRS/pressure sensor.

References

[1] F. Scholkmann, S. Kleiser, A. J. Metz, R. Zimmermann, J. Mata Pavia, U. Wolf, and M. Wolf, "A review on continuous wave functional near-infrared spectroscopy and imaging instrumentation and methodology," NeuroImage, vol. 85, pp. 6-27, 2014.

[2] N. Okui and E. Okada, "Wavelength dependence of crosstalk in dual-wavelength measurement of oxy- and deoxy-hemoglobin," Journal of Biomedical Optics, vol. 10, no. 1, p. 011015, 2005.

[3] Quandt Brit M ET AL: "Flexible POFF sensors for decubitus prevention", 1 January 2015 (2015-01-01)URL:http://www.nanotera.ch/pdf/posters2015/ParaTex187.pdf

4] Quandt Brit Maike: "Optical Fibre Textiles in Non-Invasive Medical Applications", 1 January 2016 (2016-01-01), DOI: 10.3929/ethz-a-01085260URL:https://www.research-collection.ethz.ch/handle/20.500.11850/156225

**Claims**

1. A medical device (1) for determining an oxygen saturation in a tissue of a subject, comprising:

   - a sensor (2) comprising a flexible textile (10) comprising a plurality of first optical fibers (11) and a plurality of second optical fibers (12), wherein the first optical and second optical fibers (11, 12) each comprise a first end (11a, 12a) and a second end (11b, 12b),
   - wherein the sensor (2) further comprises a first light generating unit (3) configured to generate and couple light of a first wavelength into the first ends (11a) of the first optical fibers (11) and to generate and couple light of a second wavelength into the second ends (11b) of the first optical fibers (11), the first wavelength being different from the second wavelength, wherein the respective first optical fiber (11) is configured to emit light via a light emitting section (21) of the respective first optical fiber (11) to irradiate tissue of the subject, the light emitting section (21) being arranged between the first end (11a) and the second end (11b) of the respective first optical fiber (11), and wherein the respective second optical fiber (12) comprises a light receiving section (22) configured to receive light coming from the tissue, the light receiving section (22) being arranged between the first end (12a) and the second end (12b) of the respective second optical fiber (12),
   - wherein the sensor (2) further comprises a first light detector unit (4) operatively connected to the second optical fibers (12) and configured to detect light received by the light receiving section (22) of the respective second optical fiber (12),
   - an analyzing unit (5) configured to determine an oxygen saturation value of the tissue using intensities of light detected by the first light detector unit (4).

2. The medical device according to claim 1, wherein the light emitting sections (21) of the first fibers (11) are arranged to form a plurality of separate light emitting regions (30) on the flexible textile (10), each light emitting region (30) comprising a plurality of light emitting sections (21) of a corresponding a number of first fibers (11), and/or wherein the light receiving sections (22) of the second fibers (12) are arranged to form a plurality of separate light receiving regions on (40) the flexible textile (10), each light receiving region (40) comprising a plurality of light receiving sections (22) of a corresponding number of second fibers (12).

3. The medical device according to claim 2, wherein a distance between a light emitting region (30) and a light receiving region (40) is at least 5mm, particularly at least 10 mm, particularly at least 20 mm, and/or wherein a distance between any light emitting region (30) and light receiving region (40) is in the range from 8 mm to 60 mm, and/or wherein a diameter of each light emitting region (30) is in the range from 3 mm to 10 mm, and/or wherein a diameter of each light receiving region (40) is in the range from 3 mm to 10 mm, and/or wherein the arrangement of the light emitting regions (30) and the light receiving regions (40) on the flexible textile (10) define a plurality of distances between light emitting and light receiving regions (30, 40) which allow for a measurement volume of an underlying tissue of a person to be measured throughout the measurement volume, wherein the analyzing unit (5) is configured to determine an oxygen saturation distribution of the tissue using intensities of light detected by the first light detector unit (4), the oxygen saturation distribution assigning to each of a plurality of points in said measurement volume of the tissue an oxygen saturation value of the tissue.

4. The medical device according to one of the preceding claims, wherein the medical device (1) further comprises a control unit (6) operatively connected to the first light generating unit (3) and the first light detector unit (4), the control unit (6) configured to control generation of said light by the first light generating unit (3) and to generate first raw data indicative of intensities of light detected by the first light detector unit (4).

5. The medical device according to one of the preceding claims, wherein the flexible textile (10) is configured to be placed on a skin portion of a subject to contact the skin portion, the skin portion forming an outer surface of a volume occupied by the tissue, and/or wherein the medical device (1) comprises a garment configured to be worn by the subject, the garment comprising an inside, the flexible textile (10) forming at least a portion of said inside or being connectable to said inside.

6. The medical device according to claim 4 or according to claim 5 insofar as reference is made to claim 4, wherein the control unit (6) is configured to transmit said first raw data to the analyzing unit (5).

7. The medical device according to one of the preceding claims, wherein the light emitting section (21) of the respective first optical fiber (11) is a curved light emitting section (21) comprising a curvature adapted such that the light emitting section (21) of the respective first optical fiber (11) emits said light, and/or wherein the light receiving section (22) of the respective second optical fiber (12) is a curved light receiving section (22) comprising a curvature adapted such that the light receiving section (22) of the respective second optical fiber (12) receives said light coming from the tissue.

8. The medical device according to one of the preceding claims, wherein the light emitting section (21) of the respective first optical fiber (11) comprises one of: a loop (210), preferably at least two loops (210), preferably at least three loops (210); preferably three loops arranged in a triangular configuration; a knot; a bend; and/or wherein the light receiving section (22) of the respective second optical fiber (12) comprises one of: a loop (220), preferably at least two loops (220), preferably at least three loops (220); preferably three loops arranged in a triangular configuration; a knot; a bend.

9. The medical device according to one of the preceding claims, **characterized in that** the flexible textile (10) comprises supporting fibers (14).

10. The medical device according to claim 9, wherein the respective first optical fiber (12) is fastened to a supporting fiber (14), and/or wherein the respective second optical fiber (12) is fastened to a supporting fiber (14).

11. The medical device according to claims 8 and 9, wherein the respective loop (210) of the light emitting section (21) of the respective first optical fiber (11) is looped around a supporting fiber (14).

12. The medical device according to claims 8 and 9, wherein the respective loop (220) of the light receiving section (22) of the respective second optical fiber (12) is looped around a supporting fiber (14).

13. The medical device according to one of the preceding claims, wherein the analyzing unit (5) is configured to determine at each point a concentration of oxy-hemoglobin and a concentration of deoxy-hemoglobin in the tissue of the subject from said first raw data and to determine said oxygen saturation distribution in the tissue ($StO_2$) at each point via $StO_2 = [HbO_2]/([HbO_2] + [HHb])$, where $HbO_2$ denotes the concentration of oxy-hemoglobin at the respective point and $[HHb]$ denotes the concentration of deoxy-hemoglobin at the respective point.

14. The medical device according to claim 4 and according to one of the claims 6 to 13 insofar reference is made to claim 4, wherein the sensor (2) further comprises a plurality of third optical fibers (13), each third optical fiber (13) being preferably connected to a supporting fiber (14) of the flexible textile (10), and wherein each third optical fiber (13) comprises a first end (13a) and a second end (13b) opposite the first end, and wherein the medical device (1) further comprises:

   - a second light generating unit (7) configured to generate and couple light into each third optical fiber (13) via the respective first end (13a),
   - a second light detector unit (8) configured to detect light at the second end (13b) of the respective third optical fiber (13),

   wherein the third optical fibers (13) are arranged such that they form a plurality of crossover points (130) wherein at each crossover point (130) a third optical fiber (13) crosses another third optical fiber (13) so that a pressure applied to a crossover point (130) reduces an intensity of the light detected by the second light detector unit (8) at the second ends (13b) of the respective third optical fibers (13) forming the crossover point (130), wherein the control unit (6) is further configured to generate second raw data indicative of intensities of light detected by the second light detector unit (8), and wherein the control unit (6) is configured to transmit said second raw data to the analyzing unit (5), particularly via a wireless communication connection (6a) or a cable, wherein the analyzing unit (5) is configured to determine a pressure distribution assigning to each crossover point (130) a pressure exerted onto the respective crossover point (130) from the second raw data.

15. A method for monitoring oxygen saturation in a tissue using a medical device (1) according to one of the preceding claims, the method comprising the steps of:

   (i) Arranging the flexible textile (10) on the skin of a human or animal subject such that the light emitting sections (21) and the light receiving sections (22) are located adjacent the tissue to be monitored,
   (ii) generating light of the first wavelength and of the different second wavelength with the first light generating unit (3) controlled by the control unit (6) and coupling the light of the first wavelength into the first ends of the first optical fibers (12) and the light of the second wavelength into the second ends of the first optical fibers to irradiate the tissue with the light of the first and the second wavelength via the light emitting sections (21) of the first optical fibers (11), and detecting light received by the light receiving sections (22) of the second optical fibers (12) with the first light detector unit (4), and generating first raw data with the control unit (6), the first raw data being indicative of intensities of the light detected by the first light detector unit (4), and transmitting the first raw data to the analyzing unit (5),

(iii) determining with the analyzing unit (5) a concentration of oxy-hemoglobin ($[HbO_2]$) and a concentration of deoxy-hemoglobin ([HHb]) in the tissue from the first raw data, wherein an oxygen saturation value ($StO_2$) is determined as $StO_2 = [HbO_2]/([HbO_2] + [HHb])$, where $[HbO_2]$ denotes the concentration of oxy-hemoglobin and [HHb] denotes the concentration of deoxy hemoglobin.

**Patentansprüche**

1. Eine medizinische Vorrichtung (1) zur Bestimmung einer Sauerstoffsättigung in einem Gewebe eines Individuums, umfassend:

   - einen Sensor (2), der ein flexibles Textil (10) umfasst, das eine Vielzahl von ersten optischen Fasern (11) und eine Vielzahl von zweiten optischen Fasern (12) umfasst, wobei die ersten und zweiten optischen Fasern (11, 12) jeweils ein erstes Ende (11a, 12a) und ein zweites Ende (11b, 12b) aufweisen,
   - wobei der Sensor (2) ferner eine erste Lichterzeugungseinheit (3) umfasst, die so konfiguriert ist, dass sie Licht einer ersten Wellenlänge erzeugt und in die ersten Enden (11a) der ersten optischen Fasern (11) einkoppelt sowie Licht einer zweiten Wellenlänge erzeugt und in die zweiten Enden (11b) der ersten optischen Fasern (11) einkoppelt, wobei sich die erste Wellenlänge von der zweiten Wellenlänge unterscheidet, wobei die jeweilige erste optische Faser (11) so konfiguriert ist, dass sie Licht über einen Lichtemissionsabschnitt (21) der jeweiligen ersten optischen Faser (11) emittiert, um Gewebe des Individuums zu bestrahlen, wobei der Lichtemissionsabschnitt (21) zwischen dem ersten Ende (11a) und dem zweiten Ende (11b) der jeweiligen ersten optischen Faser (11) angeordnet ist, und wobei die jeweilige zweite optische Faser (12) einen Lichtempfangsabschnitt (22) umfasst, der so konfiguriert ist, dass er vom Gewebe kommendes Licht empfängt, wobei der Lichtempfangsabschnitt (22) zwischen dem ersten Ende (12a) und dem zweiten Ende (12b) der jeweiligen zweiten optischen Faser (12) angeordnet ist,
   - wobei der Sensor (2) ferner eine erste Lichtdetektoreinheit (4) umfasst, die mit den zweiten optischen Fasern (12) wirkverbunden und so konfiguriert ist, dass sie das von dem Lichtempfangsabschnitt (22) der jeweiligen zweiten optischen Faser (12) empfangene Licht erfasst,
   - eine Analyseeinheit (5), die so konfiguriert ist, dass sie einen Sauerstoffsättigungswert des Gewebes unter Verwendung der von der ersten Lichtdetektoreinheit (4) erfassten Lichtintensitäten bestimmt.

2. Die medizinische Vorrichtung nach Anspruch 1, wobei die Lichtemissionsabschnitte (21) der ersten Fasern (11) so angeordnet sind, dass sie eine Vielzahl separater Lichtemissionsbereiche (30) auf dem flexiblen Textil (10) bilden, wobei jeder Lichtemissionsbereich (30) eine Vielzahl von Lichtemissionsabschnitten (21) einer entsprechenden Anzahl erster Fasern (11) umfasst, und/oder wobei die Lichtempfangsabschnitte (22) der zweiten Fasern (12) so angeordnet sind, dass sie eine Vielzahl von getrennten Lichtempfangsbereichen (40) auf dem flexiblen Textil (10) bilden, wobei jeder Lichtempfangsbereich (40) eine Vielzahl von Lichtempfangsabschnitten (22) einer entsprechenden Anzahl von zweiten Fasern (12) umfasst.

3. Die medizinische Vorrichtung nach Anspruch 2, wobei der Abstand zwischen einem lichtemittierenden Bereich (30) und einem lichtempfangenden Bereich (40) mindestens 5 mm, insbesondere mindestens 10 mm, insbesondere mindestens 20 mm beträgt, und/oder wobei der Abstand zwischen einem beliebigen lichtemittierenden Bereich (30) und einem lichtempfangenden Bereich (40) im Bereich von 8 mm bis 60 mm liegt, und/oder wobei der Durchmesser jedes lichtemittierenden Bereichs (30) im Bereich von 3 mm bis 10 mm liegt, und/oder wobei der Durchmesser jedes lichtempfangenden Bereichs (40) im Bereich von 3 mm bis 10 mm liegt, und/oder wobei die Anordnung der lichtemittierenden Bereiche (30) und der lichtempfangenden Bereiche (40) auf dem flexiblen Textil (10) eine Vielzahl von Abständen zwischen lichtemittierenden und lichtempfangenden Bereichen (30, 40) definiert, die es ermöglichen, ein Messvolumen eines darunterliegenden Gewebes eines Individuums über das gesamte Messvolumen hinweg zu messen, wobei die Analyseeinheit (5) so konfiguriert ist, dass sie eine Sauerstoffsättigungsverteilung des Gewebes unter Verwendung von Lichtintensitäten bestimmt, die von der ersten Lichtdetektoreinheit (4) erfasst werden, wobei die Sauerstoffsättigungsverteilung jedem einer Vielzahl von Punkten in dem Messvolumen des Gewebes einen Sauerstoffsättigungswert des Gewebes zuordnet.

4. Die medizinische Vorrichtung nach einem der vorstehenden Ansprüche, wobei die medizinische Vorrichtung (1) ferner eine Kontrolleinheit (6) umfasst, die funktionsmäßig mit der ersten Lichterzeugungseinheit (3) und der ersten Lichtdetektoreinheit (4) verbunden ist, wobei die Kontrolleinheit (6) so konfiguriert ist, dass sie die Erzeugung des Lichts durch die erste Lichterzeugungseinheit (3) kontrolliert und erste Rohdaten erzeugt, die die von der ersten Lichtdetektoreinheit (4) erfassten Lichtintensitäten anzeigen.

5. Die medizinische Vorrichtung nach einem der vorstehenden Ansprüche, wobei das flexible Textil (10) so konfiguriert ist, dass es auf einen Hautabschnitt eines Individuums aufgelegt wird, um den Hautabschnitt zu berühren, wobei der Hautabschnitt eine Außenfläche eines vom Gewebe eingenommenen Volumens bildet, und/oder wobei die medizinische Vorrichtung (1) ein Kleidungsstück umfasst, das dazu konfiguriert ist, von dem Individuum getragen zu werden, wobei das Kleidungsstück eine Innenseite aufweist, wobei das flexible Textil (10) zumindest einen Teil der Innenseite bildet oder mit der Innenseite verbunden werden kann.

6. Die medizinische Vorrichtung nach Anspruch 4 oder nach Anspruch 5, soweit auf Anspruch 4 Bezug genommen wird, wobei die Kontrolleinheit (6) so konfiguriert ist, dass sie die ersten Rohdaten an die Analyseeinheit (5) überträgt.

7. Die medizinische Vorrichtung nach einem der vorstehenden Ansprüche, wobei der lichtemittierende Abschnitt (21) der jeweiligen ersten optischen Faser (11) ein gekrümmter lichtemittierender Abschnitt (21) ist, der eine Krümmung aufweist, die so angepasst ist, dass der lichtemittierende Abschnitt (21) der jeweiligen ersten optischen Faser (11) das Licht emittiert, und/oder wobei der lichtempfangende Abschnitt (22) der jeweiligen zweiten optischen Faser (12) ein gekrümmter lichtempfangender Abschnitt (22) ist, der eine Krümmung aufweist, die so ausgelegt ist, dass der lichtempfangende Abschnitt (22) der jeweiligen zweiten optischen Faser (12) das vom Gewebe kommende Licht empfängt.

8. Die medizinische Vorrichtung nach einem der vorstehenden Ansprüche, wobei der lichtemittierende Abschnitt (21) der jeweiligen ersten optischen Faser (11) eines der folgenden Elemente umfasst: eine Schleife (210), vorzugsweise mindestens zwei Schleifen (210), vorzugsweise mindestens drei Schleifen (210); vorzugsweise drei in einer dreieckigen Anordnung angeordnete Schleifen; einen Knoten; eine Biegung; und/oder wobei der lichtempfangende Abschnitt (22) der jeweiligen zweiten optischen Faser (12) eines der folgenden Elemente umfasst: eine Schleife (220), vorzugsweise mindestens zwei Schleifen (220), vorzugsweise mindestens drei Schleifen (220); vorzugsweise drei in einer dreieckigen Anordnung angeordnete Schleifen; einen Knoten; eine Biegung.

9. Die medizinische Vorrichtung nach einem der vorstehenden Ansprüche, **dadurch gekennzeichnet, dass** das flexible Textil (10) Trägerfasern (14) umfasst.

10. Die medizinische Vorrichtung nach Anspruch 9, wobei die jeweilige erste optische Faser (12) an einer Trägerfaser (14) befestigt ist und/oder wobei die jeweilige zweite optische Faser (12) an einer Trägerfaser (14) befestigt ist.

11. Die medizinische Vorrichtung nach den Ansprüchen 8 und 9, wobei die jeweilige Schleife (210) des lichtemittierenden Abschnitts (21) der jeweiligen ersten optischen Faser (11) um eine Trägerfaser (14) geschlungen ist.

12. Die medizinische Vorrichtung nach den Ansprüchen 8 und 9, wobei die jeweilige Schleife (220) des lichtempfangenden Abschnitts (22) der jeweiligen zweiten optischen Faser (12) um eine Trägerfaser (14) geschlungen ist.

13. Die medizinische Vorrichtung nach einem der vorstehenden Ansprüche, wobei die Analyseeinheit (5) dazu konfiguriert ist, an jedem Punkt eine Konzentration von Oxyhämoglobin und eine Konzentration von Desoxyhämoglobin im Gewebe des Individuums aus den ersten Rohdaten zu bestimmen und die Sauerstoffsättigungsverteilung im Gewebe $(StO_2)$ an jedem Punkt über $StO_2 = [HbO_2]/([HbO_2] + [HHb])$ zu bestimmen, wobei $HbO_2$ die Konzentration von Oxyhämoglobin am jeweiligen Punkt und [HHb] die Konzentration von Desoxyhämoglobin am jeweiligen Punkt bezeichnet.

14. Die medizinische Vorrichtung gemäß Anspruch 4 und gemäß einem der Ansprüche 6 bis 13, soweit auf Anspruch 4 Bezug genommen wird, wobei der Sensor (2) ferner eine Vielzahl von dritten optischen Fasern (13) umfasst, wobei jede dritte optische Faser (13) vorzugsweise mit einer Trägerfaser (14) des flexiblen Textils (10) verbunden ist, und wobei jede dritte optische Faser (13) ein erstes Ende (13a) und ein dem ersten Ende gegenüberliegendes zweites Ende (13b) umfasst, und wobei die medizinische Vorrichtung (1) ferner umfasst:

- eine zweite Lichterzeugungseinheit (7), die so konfiguriert ist, dass sie Licht erzeugt und in jede dritte optische Faser (13) über das jeweilige erste Ende (13a) einkoppelt,
- eine zweite Lichtdetektoreinheit (8), die so konfiguriert ist, dass sie Licht am zweiten Ende (13b) der jeweiligen dritten optischen Faser (13) erfasst,

wobei die dritten optischen Fasern (13) so angeordnet sind, dass sie eine Vielzahl von Kreuzungspunkten (130) bilden, wobei an jedem Kreuzungspunkt (130) eine dritte optische Faser (13) eine andere dritte optische Faser (13) kreuzt, so dass ein auf einen Kreuzungspunkt (130) ausgeübter Druck eine Intensität des von der zweiten Lichtdetektoreinheit (8) an den zweiten Enden (13b) der jeweiligen dritten optischen Fasern (13), die den Kreuzungspunkt (130) bilden, erfassten Lichts verringert, wobei die Kontrolleinheit (6) ferner dazu konfiguriert ist, zweite Rohdaten zu erzeugen, die Intensitäten des von der zweiten Lichtdetektoreinheit (8) erfassten Lichts anzeigen, und wobei die Kontrolleinheit (6) dazu konfiguriert ist, diese zweiten Rohdaten an die Analyseeinheit (5) zu übertragen, insbesondere über eine drahtlose Kommunikationsverbindung (6a) oder ein Kabel, wobei die Analyseeinheit (5) dazu konfiguriert ist, aus den zweiten Rohdaten eine Druckverteilung zu ermitteln, die jedem Kreuzungspunkt (130) einen auf den jeweiligen Kreuzungspunkt (130) ausgeübten Druck zuordnet.

15. Verfahren zur Überwachung einer Sauerstoffsättigung in einem Gewebe unter Verwendung einer medizinischen Vorrichtung (1) gemäß einem der vorstehenden Ansprüche, wobei das Verfahren die folgenden Schritte umfasst:

(i) Anordnen des flexiblen Textils (10) auf der Haut eines menschlichen oder tierischen Individuums derart, dass sich die lichtemittierenden Abschnitte (21) und die lichtempfangenden Abschnitte (22) benachbart zu dem zu überwachenden Gewebe befinden,
(ii) Erzeugen von Licht der ersten Wellenlänge und der unterschiedlichen zweiten Wellenlänge mit der ersten Lichterzeugungseinheit (3), die von der Kontrolleinheit (6) kontrolliert wird, und Einkoppeln des Lichts der ersten Wellenlänge in die ersten Enden der ersten optischen Fasern (11) und des Lichts der zweiten Wellenlänge in die zweiten Enden der ersten optischen Fasern, um das Gewebe über die lichtemittierenden Abschnitte (21) der ersten optischen Fasern (11) mit dem Licht der ersten und der zweiten Wellenlänge zu bestrahlen, und Erfassen von Licht, das von den lichtempfangenden Abschnitten (22) der zweiten optischen Fasern (12) empfangen wird, mit der ersten Lichtdetektoreinheit (4) sowie Erzeugen von ersten Rohdaten mit der Kontrolleinheit (6), wobei die ersten Rohdaten die Intensitäten des von der ersten Lichtdetektoreinheit (4) erfassten Lichts angeben, und Übertragen der ersten Rohdaten an die Analyseeinheit (5),
(iii) Bestimmen einer Konzentration von Oxyhämoglobin ([HbO$_2$]) und einer Konzentration von Desoxyhämoglobin ([HHb]) im Gewebe anhand der ersten Rohdaten mit der Analyseeinheit (5), wobei ein Sauerstoffsättigungswert (StO$_2$) als StO$_2$ = [HbO$_2$ ]/([HbO$_2$ ] + [HHb]) bestimmt wird, wobei [HbO$_2$] die Konzentration von Oxyhämoglobin und [HHb] die Konzentration von Desoxyhämoglobin bezeichnet.

## Revendications

1. Dispositif médical (1) pour déterminer une saturation en oxygène dans un tissu d'un sujet, comprenant :

- un capteur (2) comprenant un textile souple (10) comprenant une pluralité de premières fibres optiques (11) et une pluralité de deuxièmes fibres optiques (12), dans lequel les premières et deuxièmes fibres optiques (11, 12) comprennent chacune une première extrémité (11a, 12a) et une seconde extrémité (11b, 12b),
- dans lequel le capteur (2) comprend en outre un premier module de génération lumineuse (3) configuré pour générer et coupler de la lumière d'une première longueur d'onde dans les premières extrémités (11a) des premières fibres optiques (11) et pour générer et coupler de la lumière d'une seconde longueur d'onde dans les secondes extrémités (11b) des premières fibres optiques (11), la première longueur d'onde étant différente de la seconde longueur d'onde, dans lequel la première fibre optique respective (11) est configurée pour émettre de la lumière par le biais d'une section d'émission lumineuse (21) de la première fibre optique respective (11) pour irradier le tissu du sujet, la section d'émission lumineuse (21) étant agencée entre la première extrémité (11a) et la seconde extrémité (11b) de la première fibre optique respective (11), et dans lequel la deuxième fibre optique respective (12) comprend une section de réception lumineuse (22) configurée pour recevoir de la lumière provenant du tissu, la section de réception lumineuse (22) étant agencée entre la première extrémité (12a) et la seconde extrémité (12b) de la deuxième fibre optique respective (12),
- dans lequel le capteur (2) comprend en outre un premier module de détecteur lumineux (4) connecté de manière opérationnelle aux deuxièmes fibres optiques (12) et configuré pour détecter de la lumière reçue par la section de réception lumineuse (22) de la deuxième fibre optique respective (12),

- un module d'analyse (5) configuré pour déterminer une valeur de saturation en oxygène du tissue en utilisant des intensités de lumière détectée par le premier module de détecteur lumineux (4).

2. Dispositif médical selon la revendication 1, dans lequel les sections d'émission lumineuse (21) des premières fibres (11) sont agencées pour former une pluralité de régions d'émission lumineuse séparées (30) sur le textile souple (10), chaque région d'émission lumineuse (30) comprenant une pluralité de sections d'émission lumineuse (21) d'un nombre correspondant de premières fibres (11), et/ou dans lequel les sections de réception lumineuse (22) des deuxièmes fibres (12) sont agencées pour former une pluralité de régions de réception lumineuse séparées (40) sur le textile souple (10), chaque région de réception lumineuse (40) comprenant une pluralité de sections de réception lumineuse (22) d'un nombre correspondant de deuxièmes fibres (12).

3. Dispositif médical selon la revendication 2, dans lequel une distance entre une région d'émission lumineuse (30) et une région de réception lumineuse (40) est d'au moins 5 mm, notamment d'au moins 10 mm, notamment d'au moins 20 mm, et/ou dans lequel une distance entre toute région d'émission lumineuse (30) et région de réception lumineuse (40) est dans la plage de 8 mm à 60 mm, et/ou dans lequel un diamètre de chaque région d'émission lumineuse (30) est dans la plage de 3 mm à 10 mm, et/ou dans lequel un diamètre de chaque région de réception lumineuse (40) est dans la plage de 3 mm à 10 mm, et/ou dans lequel l'agencement des régions d'émission lumineuse (30) et des régions de réception lumineuse (40) sur le tissu souple (10) définissent une pluralité de distances entre des régions d'émission lumineuse et de réception lumineuse (30, 40) qui permettent un volume de mesure d'un tissu sous-jacent d'une personne devant être mesuré d'un bout à l'autre du volume de mesure, dans lequel le module d'analyse (5) est configuré pour déterminer une distribution de saturation en oxygène du tissu en utilisant des intensités de lumière détectée par le premier module de détecteur lumineux (4), la distribution de saturation en oxygène attribuant à chacun d'une pluralité de points dans ledit volume de mesure du tissu une valeur de saturation en oxygène du tissu.

4. Dispositif médical selon une des revendications précédentes, dans lequel le dispositif médical (1) comprend en outre un module de commande (6) connecté de manière opérationnelle au premier module de génération lumineuse (3) et au premier module de détecteur lumineux (4), le module de commande (6) étant configuré pour commander la génération de ladite lumière par le premier module de génération lumineuse (3) et pour générer des premières données brutes indicatrices d'intensités de lumière détectée par le premier module de détecteur lumineux (4).

5. Dispositif médical selon une des revendications précédentes, dans lequel le textile souple (10) est configuré pour être placé sur une portion de peau d'un sujet pour venir en contact avec la portion de peau, la portion de peau formant une surface externe d'un volume occupé par le tissu, et/ou dans lequel le dispositif médical (1) comprend un vêtement configuré pour être porté par le sujet, le vêtement comprenant un intérieur, le textile souple (10) formant au moins une portion dudit intérieur ou pouvant être connecté audit intérieur.

6. Dispositif médical selon la revendication 4 ou selon la revendication 5 dans la mesure où il est fait référence à la revendication 4, dans lequel le module de commande (6) est configuré pour transmettre lesdites premières données brutes au module d'analyse (5).

7. Dispositif médical selon une des revendications précédentes, dans lequel la section d'émission lumineuse (21) de la première fibre optique respective (11) est une section d'émission lumineuse incurvée (21) comprenant une courbure adaptée de sorte que la section d'émission lumineuse (21) de la première fibre optique respective (11) émet ladite lumière, et/ou dans lequel la section de réception lumineuse (22) de la deuxième fibre optique respective (12) est une section de réception lumineuse incurvée (22) comprenant une courbure adaptée de sorte que la section de réception lumineuse (22) de la deuxième fibre optique respective (12) reçoit ladite lumière provenant du tissu.

8. Dispositif médical selon une des revendications précédentes, dans lequel la section d'émission lumineuse (21) de la première fibre optique respective (11) comprend un élément parmi : une boucle (210), de préférence au moins deux boucles (210), de préférence au moins trois boucles (210) ; de préférence trois boucles agencées en une configuration triangulaire ; un nœud ; un coude ; et/ou dans lequel la section de réception lumineuse (22) de la deuxième fibre optique respective (12) comprend un élément parmi : une boucle (220), de préférence au moins deux boucles (220), de préférence au moins trois boucles (220) ; de préférence trois boucles agencées en une configuration triangulaire ; un nœud ; un coude.

9. Dispositif médical selon une des revendications précédentes, **caractérisé en ce que** le tissu souple (10) comprend des fibres de support (14).

10. Dispositif médical selon la revendication 9, dans lequel la première fibre optique respective (12) est attachée à une fibre de support (14), et/ou dans lequel la deuxième fibre optique respective (12) est attachée à une fibre de support (14).

11. Dispositif médical selon les revendications 8 et 9, dans lequel la boucle respective (210) de la section d'émission lumineuse (21) de la première fibre optique respective (11) est bouclée autour d'une fibre de support (14).

12. Dispositif médical selon les revendications 8 et 9, dans lequel la boucle respective (220) de la section de réception lumineuse (22) de la deuxième fibre optique respective (12) est bouclée autour d'une fibre de support (14).

13. Dispositif médical selon une des revendications précédentes, dans lequel le module d'analyse (5) est configuré pour déterminer à chaque point une concentration en oxyhémoglobine et une concentration en désoxyhémoglobine dans le tissu du sujet à partir desdites premières données brutes et pour déterminer ladite distribution de saturation en oxygène dans le tissu ($StO_2$) à chaque point par le biais de $StO_2 = [HbO_2]/([HbO_2] + [HHb])$, où $HbO_2$ désigne la concentration en oxyhémoglobine au point respectif et [HHb] désigne la concentration en désoxyhémoglobine au point respectif.

14. Dispositif médical selon la revendication 4 et selon une des revendications 6 à 13 dans la mesure où il est fait référence à la revendication 4, dans lequel le capteur (2) comprend en outre une pluralité de troisièmes fibres optiques (13), chaque troisième fibre optique (13) étant de préférence connectée à une fibre de support (14) du tissu souple (10), et dans lequel chaque troisième fibre optique (13) comprend une première extrémité (13a) et une seconde extrémité (13b) à l'opposé de la première extrémité, et dans lequel le dispositif médical (1) comprend en outre :

 - un second module de génération lumineuse (7) configuré pour générer et coupler de la lumière dans chaque troisième fibre optique (13) par le biais de la première extrémité respective (13a),
 - un second module de détecteur lumineux (8) configuré pour détecter de la lumière à la seconde extrémité (13b) de la troisième fibre optique respective (13),

dans lequel les troisièmes fibres optiques (13) sont agencées de sorte qu'elles forment une pluralité de points de croisement (130), dans lequel à chaque point de croisement (130), une troisième fibre optique (13) croise une autre troisième fibre optique (13) de sorte qu'une pression appliquée au point de croisement (130) réduit une intensité de la lumière détectée par le second module de détecteur lumineux (8) aux secondes extrémités (13b) des troisièmes fibres optiques respectives (13) formant le point de croisement (130), dans lequel le module de commande (6) est en outre configuré pour générer des secondes données brutes indicatrices d'intensités de lumière détectée par le second module de détecteur lumineux (8), et dans lequel le module de commande (6) est configuré pour transmettre lesdites secondes données brutes au module d'analyse (5), notamment par le biais d'une connexion de communication sans fil (6a) ou d'un câble, dans lequel le module d'analyse (5) est configuré pour déterminer une distribution de pression attribuant à chaque point de croisement (130) une pression exercée sur le point de croisement respectif (130) à partir des secondes données brutes.

15. Procédé de surveillance de saturation en oxygène dans un tissu utilisant un dispositif médical (1) selon une des revendications précédentes, le procédé comprenant les étapes consistant à :

 (i) agencer le textile souple (10) sur la peau d'un sujet humain ou animal de sorte que les sections d'émission lumineuse (21) et les sections de réception lumineuse (22) sont situées de manière adjacente au tissu devant être surveillé,
 (ii) générer de la lumière de la première longueur d'onde et de la seconde longueur d'onde différente avec le premier module de génération lumineuse (3) commandé par le module de commande (6) et coupler la lumière de la première longueur d'onde dans les premières extrémités des premières fibres optiques (12) et la lumière de la seconde longueur d'onde dans les secondes extrémités des premières fibres optiques pour irradier le tissu avec la lumière de la première et la seconde longueur d'onde par le biais des sections d'émission lumineuse (21) des premières fibres optiques (11), et détecter de la lumière reçue par les sections de réception lumineuse (22) des deuxièmes fibres optiques (12) avec le premier module de détecteur lumineux (4), et générer des premières données brutes avec le module de commande (6), les premières données brutes étant indicatrices d'intensités de la lumière détectée par le premier module de détecteur lumineux (4), et transmettre les premières données brutes au module d'analyse (5),
 (iii) déterminer avec le module d'analyse (5) une concentration en oxyhémoglobine ($[HbO_2]$) et une concentration en désoxyhémoglobine ([HHb]) dans le tissu à partir des premières données brutes, dans lequel une valeur de

saturation en oxygène (StO$_2$) est déterminée en tant que StO$_2$ = [HbO$_2$]/([HbO$_2$] + [HHb]), où [HbO$_2$] désigne la concentration en oxyhémoglobine et [HHb] désigne la concentration en désoxyhémoglobine.

Fig. 1

Fig. 2

a)

1 cm

1 cm

1 cm

b)

0.5 cm

0.5 cm

0.5 cm

c)

5 - 7 cm

5 - 7 cm

Fig. 3

Fig. 4

Fig. 5

Fig. 6

a)

b)

Fig. 7

Fig. 8

a)

✳ Source Point  30
◯ Detector Point  40

length (mm)

width (mm)

Textile Sensor Area
10

b)

Skin

Fat

Muscle

Fig. 9

a)

40    30    10

| * | Sources |
|---|---------|
| ● | Detectors |

b)

13    130

c)

Fig. 10

Fig. 11

Fig. 12

a)

| Wavelength (nm) Group 1 |
|---|
| 690 |
| 740 |
| 810 |
| 880 |

| Wavelength (nm) Group 2 |
|---|
| 660 |
| 690 |
| 880 |
| 910 |

b)

✳ Source Point
◯ Detector Point

Textile S

Fig. 13

a)

## Chromophores

$O_2Hb$

HHb

$H_2O$

Lipid

600 ·········· 1000 nm

b)

## Finite Element Mesh Geometry

30mm

100mm

100mm

Skin

Fat

Muscle

Hypoxic Volume

c)

## Variable Parameters

Fat layer thickness (ATT)
[0:6]mm

Hypoxic diameter
[3:27]mm

Hypoxic degree
[0.1:0.1:1]

$StO_2^{Hypoxia} = StO_2^{Baseline}(1\text{-Hypo}°)$

Fig. 14

a)

b)

Fig. 15

Fig. 16

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **F. SCHOLKMANN** ; **S. KLEISER** ; **A. J. METZ** ; **R. ZIMMERMANN** ; **J. MATA PAVIA** ; **U. WOLF** ; **M. WOLF**. A review on continuous wave functional near-infrared spectroscopy and imaging instrumentation and methodology. *Neurolmage*, 2014, vol. 85, 6-27
- **N. OKUI** ; **E. OKADA**. Wavelength dependence of crosstalk in dual-wavelength measurement of oxy- and deoxy-hemoglobin. *Journal of Biomedical Optics*, 2005, vol. 10 (1), 011015

- **QUANDT BRIT M et al.** *Flexible POFF sensors for decubitus prevention*, 01 January 2015, http://www. nanotera.ch/pdf/posters2015/ParaTex187.pdf
- **QUANDT BRIT MAIKE**. *Optical Fibre Textiles in Non-Invasive Medical Applications*, 01 January 2016, https://www.research-collection.ethz.ch/handle/20.500.11850/156225